# EUROPEAN PATENT APPLICATION

(11) **EP 4 414 362 A1**
(43) Date of publication of application: **14.08.2024**
(21) Application number: 22878513.5
(22) Date of filing: 04.10.2022
(51) Int. Cl.: C07D 401/04, C07D 213/40, A61K 31/63

(54) **METHOD FOR PRODUCING COMPOUND OR PHARMACEUTICALLY ACCEPTABLE SALT THEREOF**

(30) Priority: 05.10.2021 JP 2021164171
(71) Applicant: EA Pharma Co., Ltd., Tokyo 104-0042 (JP)
(72) Inventor: MINAMIKAWA, Ryo, Tokyo 104-0042 (JP); MATSUTANI, Akira, Tokyo 104-0042 (JP); MAGATA, Yuya, Tokyo 104-0042 (JP); KAWANISHI, Shinji, Tokyo 104-0042 (JP); KAWAHIRA, Mizuki, Tokyo 104-0042 (JP)
(74) Representative: Mewburn Ellis LLP
(86) International application number: PCT/JP2022/037098
(87) International publication number: WO 2023/058645

(57) **Abstract**

Provided is a technique for improving the productivity in a method for producing a compound I or a pharmaceutically acceptable salt thereof. This method for producing the compound I comprises: a step for preparing a compound 16 solution by deprotecting a compound 10-R₃ in a solvent; and a step for adding a compound 15 to the compound 16 solution without isolating the compound 16 and synthesizing the compound I in the presence of a condensing agent.

## Description

### Technical Field

The present invention relates to a method for producing a compound or a pharmaceutically acceptable salt thereof.

### Background Art

A compound represented by the following Formula (I) (hereinafter also simply referred to as Compound I) or a pharmaceutically acceptable salt thereof has an α4 integrin inhibitory effect and is expected to be used in pharmaceutical applications and so on (International Publication No. WO2017/135472: Patent Literature 1). Patent Literature 1 describes a synthesis of Compound I in a specific method including purification with reversed-phase HPLC.

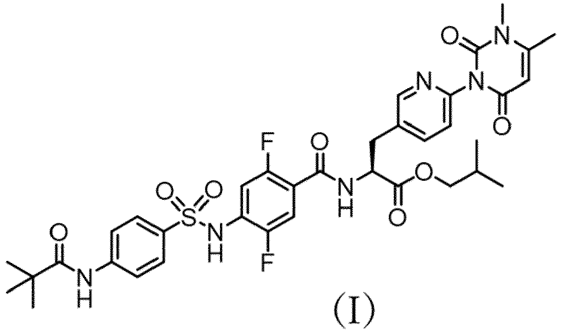

### Summary of Invention

The method for synthesizing a sulfonamide derivative represented by Compound I described in Patent Literature 1 requires column purification such as reversed-phase HPLC, and has room for further improvement in terms of productivity. Therefore, an object of the present invention is to provide a technique capable of improving the productivity in a method for producing a sulfonamide derivative represented by Compound I or a pharmaceutically acceptable salt thereof.

The present inventors found that the above object can be achieved by a specific method. Specifically, for example, the present invention provides the following matters.
(1) A method for producing a compound of the following Formula (I) or a pharmaceutically acceptable salt thereof: 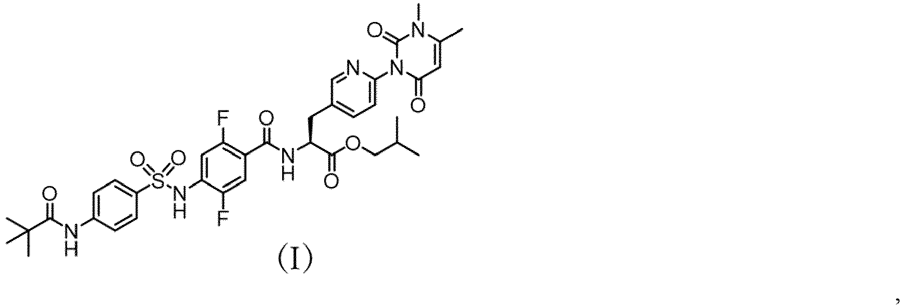 the method comprising:
   a step (d-1) of preparing a compound 16 solution by deprotecting a compound 10-R₃ in a solvent according to the following scheme (d-1): 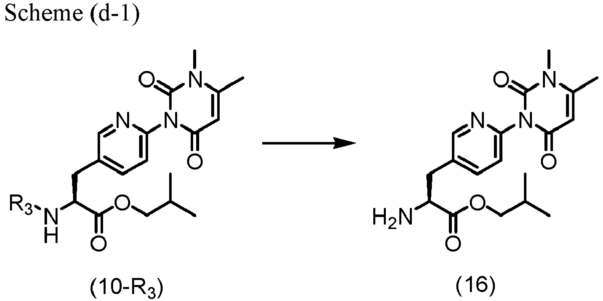 (wherein R₃ denotes a protecting group for amino group); and
   a step (d-2) of adding a compound 15 to the compound 16 solution without isolating the compound 16, and thereby synthesizing the compound of Formula (I) in the presence of a condensing agent according to the following scheme (d-2): 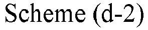 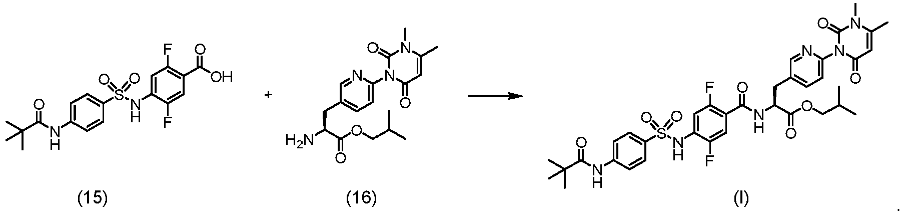
(2) The method according to (1), wherein the step (d-1) and the step (d-2) are performed in a one-pot manner.
(3) The method according to (1) or (2), wherein the step (d-1) includes a step of deprotecting the compound 10-R₃ in the presence of TMSX1 (wherein X1 denotes a halogen atom or triflate).
(4) The method according to (2) or (3), wherein the solvent in the step (d-1) includes at least one selected from the group consisting of ketone solvents, nitrile solvents, and halogenated solvents.
(5) The method according to (1), wherein the step (d-1) includes a step of deprotecting the compound 10-R₃ through catalytic reduction.
(6) The method according to (5), wherein
   the step of deprotecting through catalytic reduction includes a step of deprotecting the compound 10-R₃ in the presence of a catalyst,
   the method for producing further comprises a step of removing the catalyst from the compound 16 solution, and
   in the step (d-2), the compound 15 is added to the compound 16 solution from which the catalyst has been removed.
(7) The method according to (5) or (6), wherein the solvent in the step (d-1) includes tetrahydrofuran.
(8) The method according to any one of (1) to (7), further comprising a step (e) of crystallizing the compound of Formula (I).
(9) The method according to (8), wherein the step (e) of crystallizing includes a step of crystallizing the compound of Formula (I) in a solvent including at least one selected from the group consisting of water, alcohols having 1 to 4 carbon atoms, acetic acid esters having 3 to 6 carbon atoms, ether solvents having 2 to 10 carbon atoms, and aromatic hydrocarbons.
(10) The method according to (8) or (9), wherein the step (e) of crystallizing includes a step of crystallizing the compound of Formula (I) in a solvent including isopropanol.
(11) The method according to any one of (8) to (10), further comprising a step of drying the crystallized compound of Formula (I).
(12) The method according to any one of (1) to (11), further comprising a step (b-2) of synthesizing the compound 10-R₃ by a reaction of a compound 5-X with a compound 9-R₃ according to the following scheme (b-2): 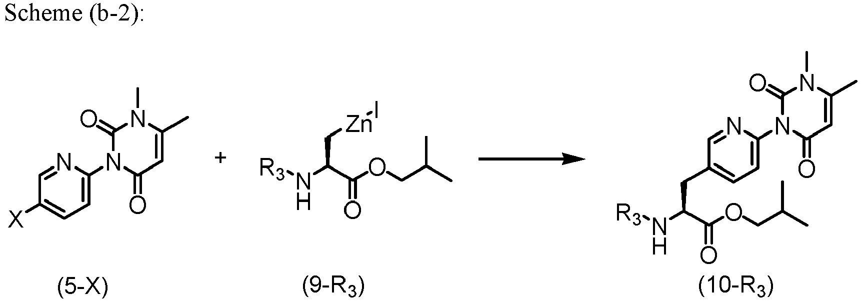 (wherein X denotes a halogen atom or triflate and R₃ denotes a protecting group for amino group).
(13) The method according to (12), wherein X is a bromine atom.
(14) The method according to (12) or (13), further comprising, before the step (b-2), a step (b-1) of synthesizing the compound 9-R₃ by a reaction of a compound 8-R₃ with zinc and iodine according to the following scheme (b-1): 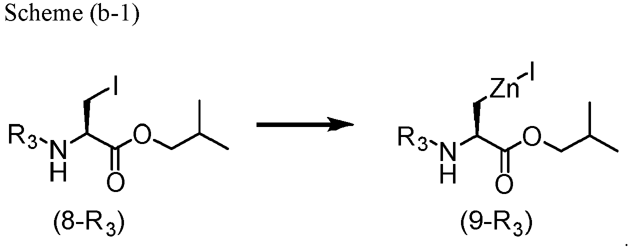
(15) The method according to any one of (12) to (14), further comprising a step (a-1) of synthesizing the compound 5-X according to the following scheme (a-1): 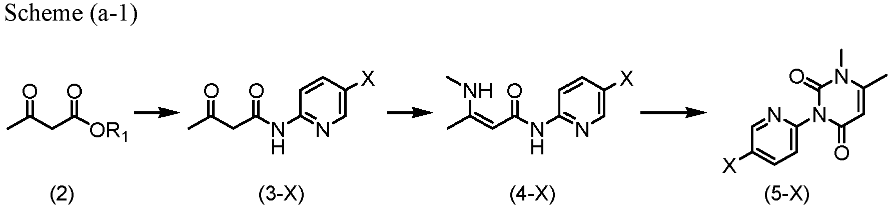 (wherein X denotes a halogen atom or triflate and R₁ denotes an alkyl group having 1 to 6 carbon atoms), wherein
   the step (a-1) includes
   a step of synthesizing a compound 3-X by a reaction of a compound 2 with a 2-aminopyridine derivative,
   a step of synthesizing a compound 4-X by a reaction of the compound 3-X with methylamine, and
   a step of synthesizing the compound 5-X by a reaction of the compound 4-X with a chloroformic acid ester.
(16) The method according to any one of (12) to (14), further comprising a step
   (a-2) of synthesizing the compound 5-X according to the following scheme (a-2): 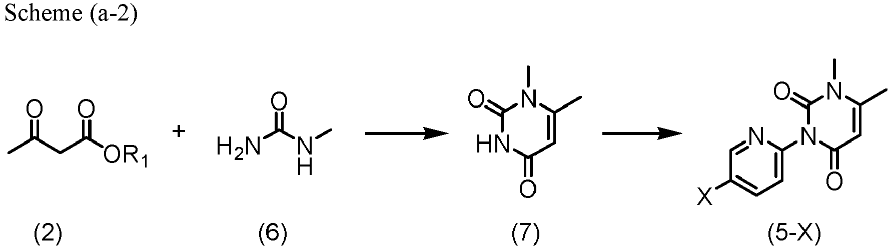 (wherein X denotes a halogen atom or triflate and R₁ denotes an alkyl group having 1 to 6 carbon atoms), wherein
      the step (a-2) includes
      a step of synthesizing a compound 7 by a reaction of a compound 2 with a compound 6, and
      a step of synthesizing the compound 5-X by coupling the compound 7 with a pyridine derivative.
(17) The method according to any one of (1) to (16), further comprising a step (c-3) of synthesizing the compound 15 by a reaction of a compound 13 with a compound 14-R₂ to form a sulfonamide, followed by hydrolysis with alkali according to the following scheme (c-3): 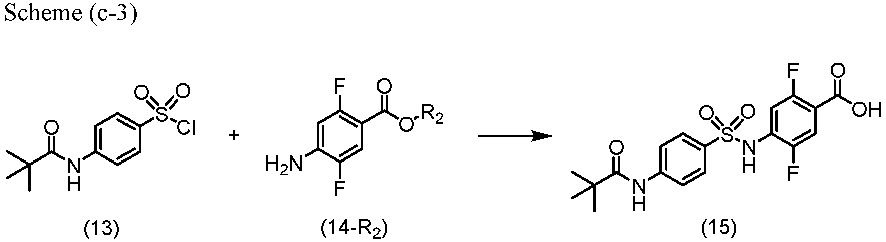 (wherein R₂ denotes an alkyl group having 1 to 6 carbon atoms).
(18) The method according to (17), further comprising, after the step (c-3), a step of crystallizing the compound 15.
(19) The method according to (17) or (18), further comprising a step of synthesizing the compound 13 by a reaction of a compound 12 with sodium hypochlorite under an acidic condition according to the following scheme (c-2): 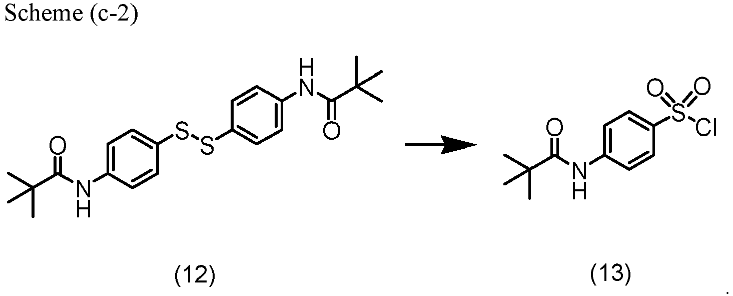
(20) The method according to (19), further comprising a step (c-1) of synthesizing the compound 12 by a reaction of a compound 11 with a pivaloylation agent according to the following scheme (c-1): 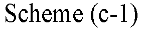 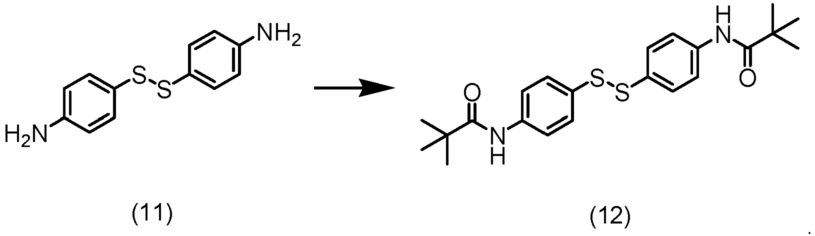
(21) A compound represented by the following formula or a salt thereof: 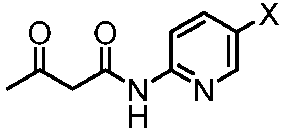 (wherein X denotes a halogen atom or triflate).
(22) A compound represented by the following formula or a salt thereof: 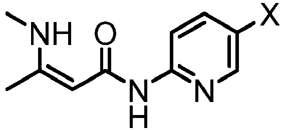 (wherein X denotes a halogen atom or triflate).
(23) A crystal of a compound of the following Formula (I) or a pharmaceutically acceptable salt thereof: 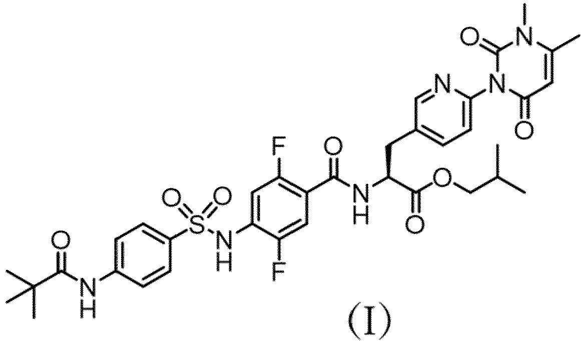
(24) The crystal according to (23), which is obtained in a method including a step of crystallizing the compound of Formula (I) in a solvent including at least one selected from the group consisting of water, alcohols having 1 to 4 carbon atoms, acetic acid esters having 3 to 6 carbon atoms, ether solvents having 2 to 10 carbon atoms, and aromatic hydrocarbons.
(25) The crystal according to (24), wherein the solvent includes isopropanol.
(26) The crystal according to (24) or (25), which is obtained by drying a crystallized product after the step of crystallizing.
(27) The crystal according to any one of (23) to (26), wherein a solubility at pH 6.9 is 50 µg/ml or more.
(28) The crystal according to any one of (23) to (27), which is a solvate with ethanol.
(29) The crystal according to any one of (23) to (27), which is a solvate with isopropanol.
(30) The crystal according to any one of (23) to (29), which is a crystal B having peaks in powder X-ray diffraction, at which a diffraction angle (2θ) is
   6.3°±0.2°,
   10.1 °±0.2°,
   13.7°±0.2°,
   16.3°±0.2°, and
   20.2°±0.2°.
(31) The crystal according to any one of (23) to (29), which is a crystal A having peaks in powder X-ray diffraction, at which a diffraction angle (2θ) is
   5.4°±0.2°,
   7.7°±0.2°,
   10.5°±0.2°,
   12.5°±0.2°, and
   14.5°±0.2°.
(32) The crystal according to any one of (23) to (29), which is a crystal C having peaks in powder X-ray diffraction, at which a diffraction angle (2θ) is
   6.8°±0.2°,
   10.1 °±0.2°,
   12.7°±0.2°,
   17.4°±0.2°, and
   19.8°±0.2°.

According to the present invention, provided is a technique capable of improving the productivity in a method for producing a sulfonamide derivative represented by Compound I or a pharmaceutically acceptable salt thereof.

### Brief Description of Drawings

Fig. 1 is a schematic diagram illustrating an entire image of a method for producing a compound I.
Fig. 2 is a diagram illustrating an X-ray diffraction pattern of a crystal A.
Fig. 3 is a diagram illustrating an X-ray diffraction pattern of a crystal B.
Fig. 4 is a diagram illustrating an X-ray diffraction pattern of a crystal C.

### Description of Embodiments

### Definitions

As used herein, "an alkyl group" means a functional group obtained by removing one hydrogen atom from an alkane (including a cycloalkane). The alkyl groups include linear, branched, and cyclic alkyl groups. Examples of the alkyl groups include a methyl group, an ethyl group, an n-propyl group, an isopropyl group, an n-butyl group, an isobutyl group, a tert-butyl group, an n-pentyl group, a neopentyl group, an n-hexyl group, and so on.

As used herein, "a halogen atom" means an atom of an element in group 17 of the periodic table. Examples of the halogen atom include a fluorine atom, a chlorine atom, a bromine atom, an iodine atom, and so on.

As used herein, "an alcohol having 1 to 4 carbon atoms" means an alcohol whose number of carbon atoms is 1 to 4. Examples of the "alcohol having 1 to 4 carbon atoms" include methanol, ethanol, isopropanol, n-butanol, isobutanol, and so on.

As used herein, "a ketone solvent" means a solvent of a compound having a ketone structure in its molecule. The ketone solvent may be acetone, methyl ethyl ketone, or the like.

As used herein, "an acetic acid ester having 3 to 6 carbon atoms" means an ester formed of acetic acid and an alcohol having 1 to 4 carbon atoms. The "acetic acid ester having 3 to 6 carbon atoms" may be ethyl acetate, propyl acetate, isopropyl acetate, isobutyl acetate, or the like.

As used herein, "an ether solvent having 2 to 10 carbon atoms" means a solvent of a compound having an ether structure in its molecule and having 2 to 10 carbon atoms. The "ether solvents having 2 to 10 carbon atoms" include linear, branched, and cyclic ether solvents. Specifically, the ether solvent having 2 to 10 carbon atoms may be 1,4-dioxane, tetrahydrofuran, 2-methyltetrahydrofuran, cyclopentyl methyl ether, tert-butyl methyl ether, or the like.

As used herein, "a nitrile solvent" means a solvent of a compound having a nitrile structure in its molecule. Examples of the nitrile solvent include acetonitrile, propionitrile, and so on.

As used herein, "a halogenated solvent" means a solvent of a compound having a halogen atom in its molecule. Examples of the halogenated solvent include dichloromethane, chloroform, and so on.

### Method for Producing Compound I

Hereinafter, a method for producing Compound I or a pharmaceutically acceptable salt thereof according to an embodiment of the present invention will be described. Compound I is a compound represented by the following Formula.

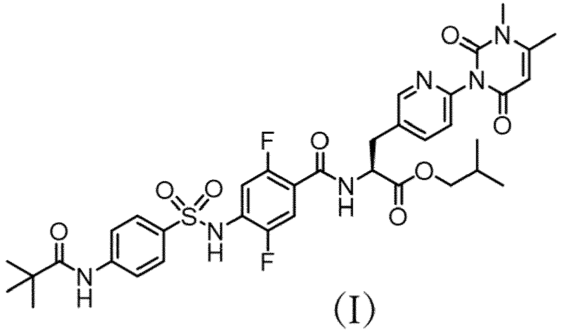

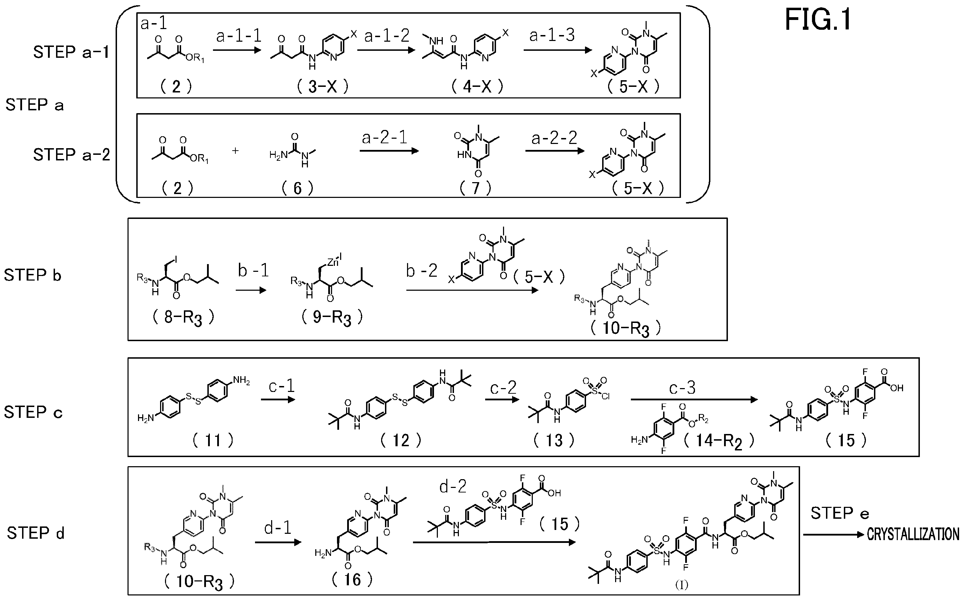

Fig. 1 illustrates an entire image of the method for producing Compound I or a pharmaceutically acceptable salt thereof according to the embodiment.

The method for producing according to the embodiment as illustrated in Fig. 1, typically includes a step of synthesizing a compound 5-X (step a), a step of synthesizing a compound 10-R₃ (step b), a step of synthesizing a compound 15 (step c), a step of synthesizing Compound I (step d), and a step of crystallizing Compound I (step e).

One of the features of the embodiment resides in the step d. In the step d in the embodiment, a compound 16 solution is prepared by deprotecting the compound 10-R₃ in a solvent (step d-1). Then, without isolating the compound 16, the compound 15 is added to the compound 16 solution to synthesize Compound I in the presence of a condensing agent (step d-2).

This method is superior to the method described in Patent Literature 1 in terms of the productivity. In the method described in Patent Literature 1, a compound corresponding to the compound 10-R₃ is deprotected by using hydrochloric acid, and the resultant solid is filtered out to obtain a hydrochloride of a compound corresponding to the compound 16 (Patent Literature 1, paragraph 0068 and so on). In other words, the solvent is removed once, and the compound corresponding to the compound 16 is isolated. Then, a compound corresponding to Compound I is synthesized by a reaction of the obtained hydrochloride of the compound with a compound corresponding to the compound 15 (Patent Literature 1, 0090, 0092, and so on). Further, the obtained compound corresponding to Compound I is purified with reversed-phase HPLC (Patent Literature 1, 0090 and so on).

Here, according to the knowledge of the present inventors, the hydrochloride of the compound 16 is deliquescent and therefore is difficult to be handled and unsuitable for mass production. In addition, the method described in Patent Literature 1 has no way to crystallize Compound I unless reversed-phase HPLC is performed after the synthesis of Compound I. The method requires Compound I to be purified through reversed-phase HPLC, which is less suitable for mass production than crystallization, and in this respect is also unsuitable for mass production.

In contrast, the method according to the embodiment is capable of synthesizing Compound I without forming the hydrochloride of the compound 16, which is difficult to be handled. As a result, the productivity can be improved.

In addition, surprisingly, the method according to the embodiment is capable of purifying the obtained Compound I through crystallization without using reversed-phase HPLC or the like. There is no need to use reversed-phase HPLC, which has a problem in the productivity. Also from this viewpoint, the productivity is improved.

Hereinafter, steps in the method for producing according to the embodiment will be described in details.

### Step a: Synthesis of Compound 5-X

This step is to synthesize the compound 5-X in a step a-1 or step a-2. Whether to use a step a-1 or step a-2 may be selected as needed. These steps will be described below.

### Step a-1

The step a-1 includes a step of synthesizing a compound 3-X by a reaction of a compound 2 with a 2-aminopyridine derivative (step a-1-1), a step of synthesizing a compound 4-X by a reaction of the compound 3-X with methylamine (step a-1-2), and a step of synthesizing a compound 5-X by a reaction of the compound 4-X with a chloroformic acid ester (step a-1-3). Hereinafter, the steps will be described.

### (Step a-1-1)

First, the compound 3-X is synthesized by the reaction of the compound 2 with a 2-aminopyridine derivative according to the following scheme.

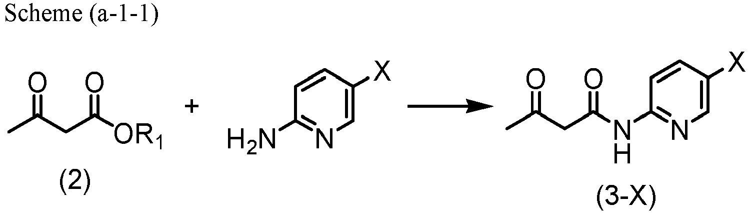

R₁ denotes an alkyl group having 1 to 6 carbon atoms. R₁ is preferably a methyl group, an ethyl group, or an n-propyl group, and is more preferably an ethyl group.

X denotes a halogen atom or triflate. The halogen atom may be a fluorine atom, a chlorine atom, a bromine atom, an iodine atom, or the like. X is preferably a bromine atom or an iodine atom, and particularly preferably a bromine atom.

The reaction can be carried out through heating to reflux in the presence of a base.

The base may be, but not particularly limited to, triethylamine, N,N-diisopropylethylamine, N-methylmorpholine, or the like, and is preferably N,N-diisopropylethylamine.

As a solvent, for example, an aromatic hydrocarbon solvent may be used. The aromatic hydrocarbon solvent may be toluene, xylene, benzene, or the like, and is preferably toluene.

The obtained compound 3-X can be precipitated as crystals by, for example, cooling the reaction liquid. The compound 3-X can be also obtained as dry crystals by drying the crystals.

### (Step a-1-2)

Subsequently, the compound 4-X is synthesized by a reaction of the compound 3-X with methylamine according to the following scheme.

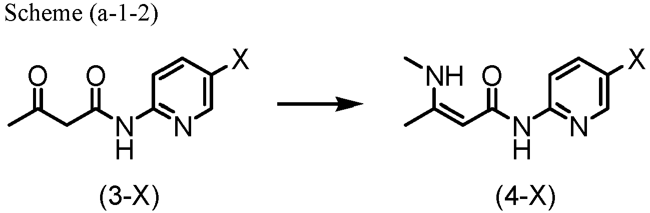

The reaction may be carried out in, for example, an alcohol having 1 to 4 carbon atoms, and preferably methanol.

The obtained compound 4-X can be precipitated as crystals by, for example, cooling the reaction liquid. The compound 4-X can be also obtained as dry crystals by drying the crystals.

### (Step a-1-3)

After that, the compound 5-X is synthesized by a reaction of the compound 4-X with a chloroformic acid ester according to the following scheme.

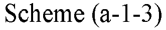

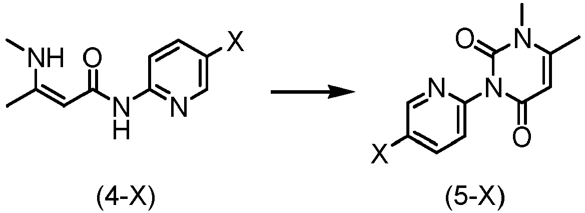

Specifically, the reaction of the compound 4-X with the chloroformic acid ester is caused in the presence of a base. After that, a strong base is further added. As a result, the compound 4-X can be converted to the compound 5-X.

The base may be triethylamine, N,N-diisopropylethylamine, N-methylmorpholine, or the like, and is preferably N,N-diisopropylethylamine.

The chloroformic acid ester may be a phenyl chloroformate, ethyl chloroformate, benzyl chloroformate, or the like, and is preferably phenyl chloroformate.

As the strong base, diazabicycloundecene or the like may be used.

A solvent used in this step may be an ether solvent having 2 to 10 carbon atoms, ethyl acetate, or toluene. The ether solvent having 2 to 10 carbon atoms may be tetrahydrofuran, 2-methyltetrahydrofuran, or the like, and is preferably tetrahydrofuran.

The obtained compound 5-X can be purified through crystallization.

A crystallization solvent may be an ether solvent having 2 to 10 carbon atoms, an alcohol having 1 to 4 carbon atoms, water, a mixture solvent thereof, or the like. Preferably, the crystallization solvent is a mixture solvent of methyl tert-butyl ether and isopropanol, a mixture solvent of methyl tert-butyl ether and isobutanol, or a mixture solvent of methyl tert-butyl ether and water. More preferably, the crystallization solvent is a mixture solvent of methyl tert-butyl ether and isopropanol or a mixture solvent of methyl tert-butyl ether and isobutanol.

Here, in order to remove impurities from the reaction liquid, it is preferable to add activated carbon or the like before the crystallization.

The compound 5-X can be obtained in the method described above.

According to the above method (step a-1), it is possible to obtain the compound 5-X at high yield and high purity with less generation of isomers. In addition, there is no need for column purification because the purification can be done through the crystallization. The above method can achieve high productivity because of no need for column purification.

### Step a-2

Next, the step a-2 will be described. The step a-2 includes a step of synthesizing a compound 7 from the compound 2 and a compound 6 (step a-2-1) and a step of synthesizing the compound 5-X by coupling the compound 7 with a pyridine derivative (step a-2-2). These steps will be described below in details.

### (Step a-2-1)

First, the compound 2 and the compound 6 are heated to reflux in the presence of an acidic catalyst to synthesize the compound 7 according to the following scheme.

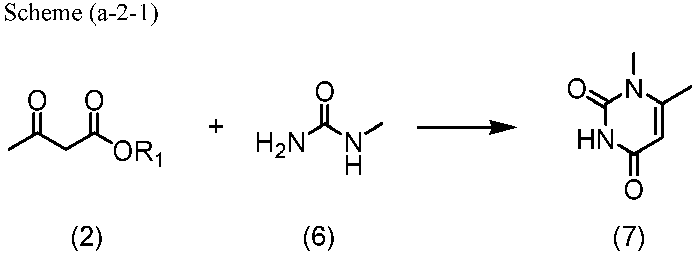

Here, R₁ denotes an alkyl group having 1 to 6 carbon atoms. R₁ is preferably a methyl group, an ethyl group, or an n-propyl group and more preferably an ethyl group.

The acidic catalyst may be sulfuric acid, p-toluenesulfonic acid monohydrate, or the like, and is preferably p-toluenesulfonic acid monohydrate.

A solvent may be an aromatic hydrocarbon solvent. The aromatic hydrocarbon solvent may be toluene, xylene, benzene, or the like, and is preferably toluene.

The obtained compound 7 can be precipitated as crystals by, for example, cooling the reaction liquid. The compound 7 can be also obtained as dry crystals by drying the crystals.

### (Step a-2-2)

Subsequently, the compound 5-X is synthesized by coupling the compound 7 with a pyridine derivative according to the following scheme.

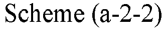

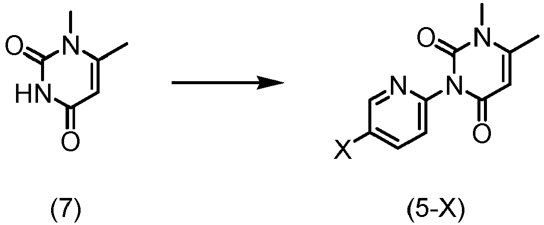

The above coupling reaction can be carried out by using a compound containing copper in the presence of a base.

As the pyridine derivative, for example, 2,5-dibromopyridine or the like may be used.

X denotes a halogen atom or triflate. The halogen atom may be a fluorine atom, a chlorine atom, a bromine atom, an iodine atom, or the like. X is preferably a bromine atom or an iodine atom, and particularly preferably a bromine atom.

The base may be 2,4,6-collidine, diazabicycloundecene, or the like, and is preferably 2,4,6-collidine.

The compound containing copper may be copper(I) oxide, copper iodide, or the like, and is preferably copper(I) oxide.

A solvent may be acetonitrile, N,N-dimethylformamide, or the like, and is preferably N,N-dimethylformamide.

The obtained compound 5-X can be purified through crystallization. A crystallization solvent is preferably an ether solvent having 2 to 10 carbon atoms, and is particularly preferably methyl tert-butyl ether.

Also according to the step a-2 described above, the compound 5-X can be obtained without column purification as in the step a-1. The above method can achieve an improved productivity because of no need for column purification.

However, the step a-1 is capable of synthesizing the compound 5-X at higher yield than the step a-2 does. In this respect, the step a-1 can be said to be a method superior to the step a-2.

### Step b: Synthesis of Compound 10

Next, the step b will be described. The step b includes a step of synthesizing a compound 9-R₃ from the compound 8-R₃ (step b-1) and a step of synthesizing a compound 10-R₃ by a reaction of the compound 9-R₃ with the compound 5-X (step b-2). These steps will be described below.

### (Step b-1)

First, the compound 9-R₃ is synthesized by a reaction of the compound 8-R₃ with zinc and iodine according to the following scheme (b-1).

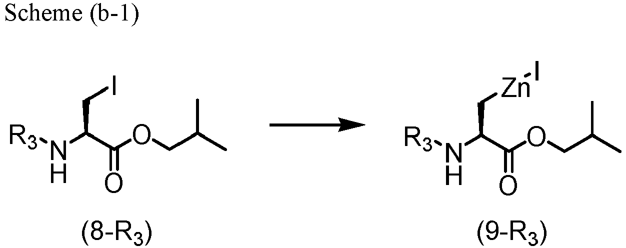

Here, R₃ denotes a protecting group for amino group.

For example, the compound 9-R₃ can be obtained by adding zinc, iodine, and the compound 8-R₃ to N,N-dimethylformamide, followed by stirring.

R₃ may be any protecting group without particular limitation, as long as the protecting group will not be detached during the reaction in the step b-1. Examples of R₃ include a tert-butoxycarbonyl group (Boc group), a benzyloxycarbonyl group (Z group or Cbz group), an allyloxycarbonyl group (Alloc group), a 2,2,2-trichloroethoxycarbonyl group (Troc group), and the like. Preferably, R₃ is a tert-butoxycarbonyl group (Boc group) or a benzyloxycarbonyl group (Z group or Cbz group). More preferably, R₃ is a tert-butoxycarbonyl group (Boc group).

### (Step b-2)

Subsequently, the compound 10-R₃ is synthesized by a reaction of the compound 5-X with the compound 9-R₃ according to the following scheme (b-2).

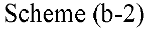

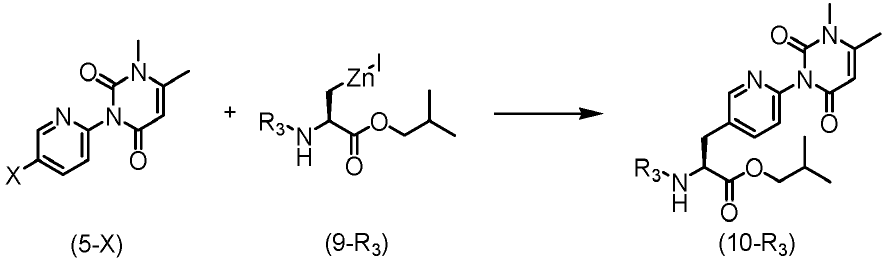

Specifically, the compound 9-R₃ is added to a solution in which the compound 5-X, a phosphine ligand, and a palladium catalyst are mixed. As a result, the conversion to the compound 10-R₃ can be achieved.

As the phosphine ligand, for example, S-Phos may be used.

As the palladium catalyst, for example, palladium acetate may be used.

A solvent may be N,N-dimethylformamide, toluene, 2-methyltetrahydrofuran, N-methyl-2-pyrrolidone, a mixture solvent thereof, or the like, and is preferably N,N-dimethylformamide.

The compound 10-R₃ can be purified through crystallization. A crystallization solvent may be, for example, an ether solvent having 2 to 10 carbon atoms. Preferably, methyl tert-butyl ether is used.

According to the method described above, the compound 10-R₃ can be obtained. According to the method with this step b, it is possible to purify the compound 10-R₃ through crystallization. As far as the present inventors know, any synthesis method capable of purifying the compound 10-R₃ through crystallization has not been known. In contrast, according to the method in the embodiment, the compound 10-R₃ can be purified with no need for column purification. Therefore, the compound 10-R₃ at high purity can be obtained by the highly productive method.

### Step c: Synthesis of Compound 15

Subsequently, a compound 15 is synthesized in the step c. The step c is achieved through steps c-1 to c-3. These steps will be described below in details.

### (Step c-1)

In the step c-1, a compound 12 is synthesized by a reaction of a compound 11 with a pivaloylation agent according to the following scheme (c-1).

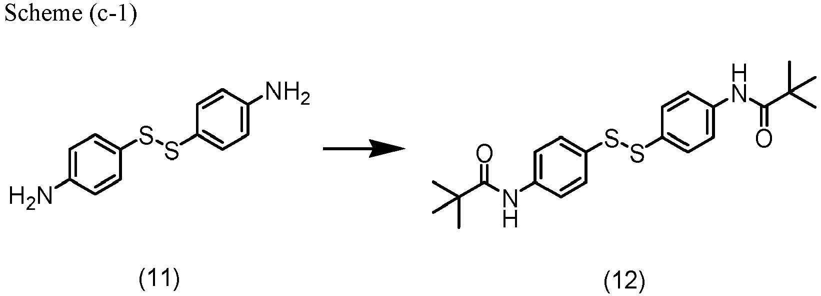

Specifically, the amino groups in the compound 11 are pivaloylated by a reaction of the compound 11 with the pivaloylation agent in the presence of a base.

The pivaloylation agent may be, for example, pivaloyl chloride, pivaloyl bromide, pivaloyl fluoride, pivalic anhydride, or the like, and is preferably pivaloyl chloride.

The base may be triethylamine, N,N-diisopropylethylamine, N-methylmorpholine, or the like, and is preferably triethylamine.

A solvent may be dichloromethane or the like.

### (Step c-2)

Next, a compound 13 is synthesized by oxidizing the compound 12 under an acidic condition according to the following scheme (c-2).

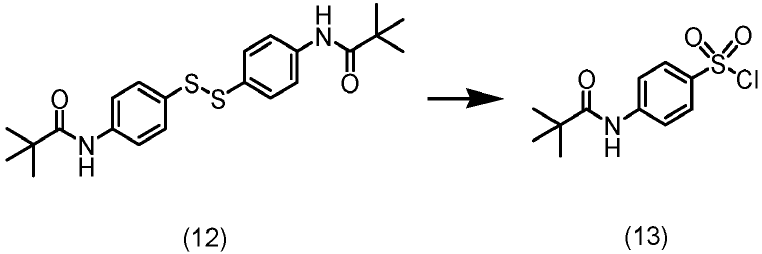

Specifically, the compound 13 is synthesized by a reaction of the compound 12 with an oxidizing agent in an acidic solvent.

As the acidic solvent, for example, acetic acid may be used.

As the oxidizing agent, for example, sodium hypochlorite may be used.

Here, crystals of the compound 13 may be precipitated by cooling the reaction liquid. Dry crystals of the compound 13 can be also obtained by drying the precipitated crystals.

### (Step c-3)

Then, the compound 15 is synthesized by a reaction of the compound 13 with a compound 14-R₂ according to the following scheme (c-3).

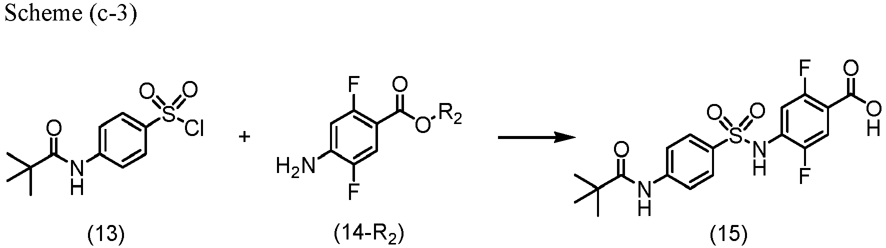

Here, R₂ denotes an alkyl group having 1 to 6 carbon atoms. R₂ may be a methyl group, an ethyl group, an n-propyl group, an isopropyl group, an n-butyl group, an n-hexyl group, or the like, and is preferably a methyl group.

### (Step c-3-1)

Specifically, first, a compound 15-R₂ is synthesized by forming a sulfonamide from the compound 14-R₂ using the compound 13 according to the following scheme.

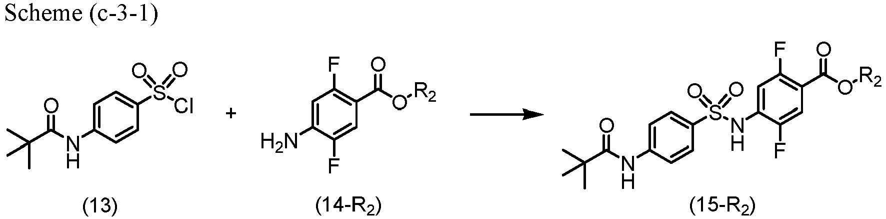

More specifically, the compound 13, the compound 14-R₂, and a base are mixed in a solvent. As a result, the compound 15-R₂ is obtained.

The base may be pyridine, triethylamine, N,N-diisopropylethylamine, N-methylmorpholine, or the like, and is preferably pyridine.

The solvent is preferably dichloromethane.

Then, the compound 15-R₂ is converted to the compound 15 according to the following scheme.

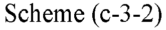

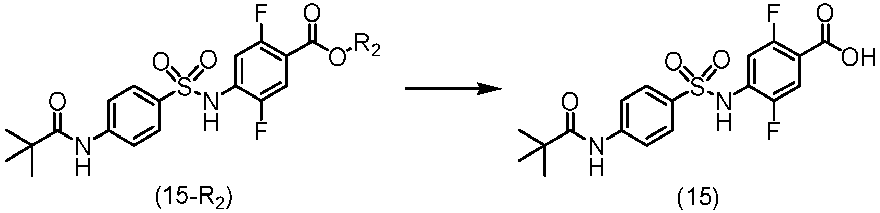

Specifically, the compound 15 can be obtained by an ester hydrolysis reaction on the compound 15-R₂ under alkali conditions.

Examples of an alkaline substance for making the reaction system alkaline conditions include a lithium hydroxide aqueous solution, a sodium hydroxide aqueous solution, a potassium hydroxide aqueous solution, and so on. The alkaline substance is preferably a sodium hydroxide aqueous solution.

A solvent may be an alcohol having 1 to 4 carbon atoms, an ether solvent having 2 to 10 carbon atoms, or a mixture solvent thereof. As the solvent, a mixture solvent of methanol and tetrahydrofuran is preferably used.

The compound 15 can be purified at high purity through crystallization. A crystallization solvent may be an alcohol having 1 to 4 carbon atoms, an ether solvent having 2 to 10 carbon atoms, a ketone solvent, a mixture solvent thereof, or the like. As the crystallization solvent, a mixture solvent of tetrahydrofuran, acetone, and water is preferably used.

According to the step c described above, the compound 15 can be obtained at high yield and high purity in the highly productive method because of no need for column purification.

### Step d: Synthesis of Compound I

Next, Compound I is synthesized in the step d. Specifically, a compound 16 solution is prepared by deprotecting the compound 10-R₃ in a solvent (step d-1) according to a scheme (d-1).

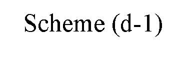

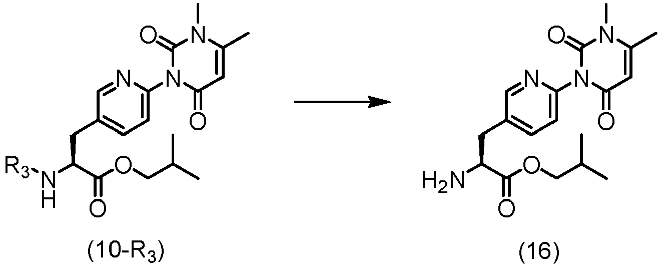

Subsequently, without isolating the compound 16, the compound 15 is added to the compound 16 solution to synthesize the compound of Formula (I) in the presence of a condensing agent (step d-2) according to the following scheme.

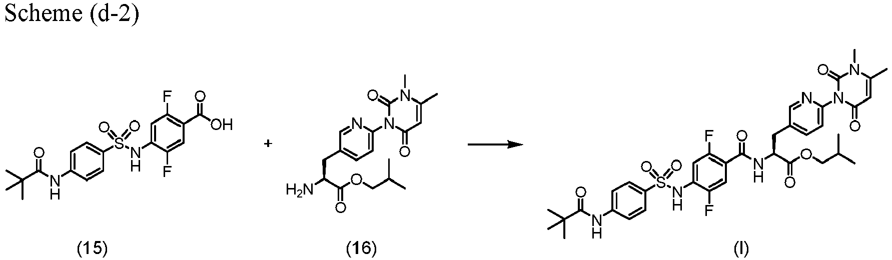

Here, to add the compound 15 to the compound 16 solution means to input a substance necessary for the reaction in the step (d-2) such as the compound 15 to the compound 16 solution without removing the solvent after the compound 16 solution is obtained in step (d-1).

The above method is capable of synthesizing Compound I without using a hydrochloride of the compound 16, which is difficult to be handled, as described above. As a result, the productivity can be improved. In addition, surprisingly, the method according to the embodiment is capable of purifying Compound I thus synthesized through crystallization. Since there is no need to use reversed-phase HPLC, which has a problem in the productivity, the productivity is improved from this viewpoint.

As examples of a specific method for the step d, there are a one-pot method and a catalytic reduction method to be described below. These methods will be described below.

### <One-Pot Method>

First, the one-pot method will be described. In the one-pot method, the step d-1 and step d-2 are performed in one pot. Specifically, the compound 16 solution obtained in the step d-1 is used in the step d-2 while being contained in the same reaction vessel.

### (1) Step (d-1) in One-Pot Method

Specifically, in the one-pot method, the compound 10-R₃ is deprotected by using TMSX1 in the step d-1. Here, TMS denotes trimethylsilyl. X1 denotes a halogen atom or triflate.

X1 in TMSX1 may be an iodine group, a bromo group, a trifluoromethyl sulfonyl group, or the like.

TMSX1 may be, for example, trimethylsilyl iodide (TMSI), trimethylsilyl bromide (TMSBr), trimethylsilyl triflate (TMSOTf), or the like, and is preferably trimethylsilyl iodide (TMSI). Trimethylsilyl iodide (TMSI) may be generated in a reaction system by using trimethylsilyl chloride (TMSCl) and sodium iodide.

A solvent may be a ketone solvent, a nitrile solvent, a halogenated solvent, or the like, and is preferably acetonitrile.

### (2) Step (d-2) in One-Pot Method

Next, the step (d-2) is performed by using the compound 16 solution obtained in the step (d-1) as it is without isolation of the compound 16. Specifically, Compound (I) is obtained through an amidation of the compound 16 by adding the compound 15, a condensing agent, a condensation aid, and a base to the compound 16 solution.

Examples of the condensing agent include 1-ethyl-3-(3-dimethylaminopropyl)carbodiimide hydrochloride (WSC hydrochloride), N,N'-dicyclohexylcarbodiimide (DCC), O-(7-azabenzotriazol-1-yl)-N,N,N',N'-tetramethyluronium hexafluorophosphate (HATU), and so on. The condensing agent is preferably 1-ethyl-3-(3-dimethylaminopropyl)carbodiimide hydrochloride (WSC hydrochloride).

The condensation aid may be 1-hydroxybenzotriazole (HOBt), 1-hydroxy-7-azabenzotriazole (HOAt), or the like, and is preferably 1-hydroxybenzotriazole (HOBt).

The base may be triethylamine, N,N-diisopropylethylamine, N-methylmorpholine, or the like, and is preferably N-methylmorpholine.

A solvent may be acetonitrile, N,N-dimethylformamide, dichloromethane, or the like, and is preferably acetonitrile.

### <Catalytic Reduction Method>

Next, the catalytic reduction method will be described.

### (1) Step (d-1) in Catalytic Reduction Method

In the catalytic reduction method, the compound 10-R₃ is deprotected through catalytic reduction using a catalyst in the step d-1.

As the catalyst, a metal catalyst is used. Preferable metals for the metal catalyst include ruthenium, rhodium, palladium, platinum, and so on. The metal catalyst is preferably palladium on carbon.

As a solvent, for example, an ether solvent having 2 to 10 carbon atoms is used and tetrahydrofuran is preferably used.

### (2) Step (d-2) in Catalytic Reduction Method

In the catalytic reduction method, the catalyst is removed from the obtained compound 16 solution. Although the catalyst is removed, a process of isolating the compound 16 from the compound 16 solution is not performed. In addition, the solvent is not removed from the compound 16 solution either. The step (d-2) is performed by using the compound 16 solution simply after the catalyst has been removed. The step (d-2) can be carried out in the same method as in the one-pot method.

### Step e: Crystallization of Compound I

Next, Compound (I) is purified through crystallization. Specifically, Compound (I) is crystallized in a crystallization solvent.

The crystallization solvent may be one selected from the group consisting of water, alcohols having 1 to 4 carbon atoms, acetic acid esters having 3 to 6 carbon atoms, ether solvents having 2 to 10 carbon atoms, and aromatic hydrocarbon. As the crystallization solvent, isopropanol is preferably used.

The crystallized compound may be dried if necessary and used according to the purpose.

The obtained crystals can be converted to another crystal form through re-crystallization in a nitrile solvent, an acetic acid ester having 3 to 6 carbon atoms, an aliphatic hydrocarbon solvent (such as hexane, heptane, or octane), or a mixture solvent thereof.

The solvent used in the re-crystallization is preferably acetonitrile, isopropyl acetate, heptane, or a mixture solvent thereof.

Compound I can be produced in the method described above. Although steps a to e are described as the method for producing Compound I in the foregoing description, each of these steps may be regarded as an independent method for producing the compound. For example, the step a-1-1 can be independently regarded as a novel and useful method for producing the compound 3-X. The same applies to the other steps.

### Crystals of Compound I

One embodiment also relates to the crystals of Compound I obtained in the foregoing method. Specifically, in the step (e), the crystals of Compound (I) can be obtained through the crystallization of Compound (I) in the crystallization solvent.

Note that amorphous Compound (I) can be obtained when the solvent is simply removed after the step (d).

With the method according to the embodiment, it is possible to obtain multiple crystal forms for Compound I. Specifically, the crystal forms include a crystal A, a crystal B, and a crystal C. These crystal forms will be described below.

### (Crystal A)

The crystal A can be obtained, for example, through crystallization of Compound (I) using ethanol or isopropanol as the crystallization solvent in the step (e) described above.

The crystal A is a crystal form having characteristic peaks in powder X-ray diffraction, at which a diffraction angle (2θ) is the following positions:
5.4°±0.2°;
10.5°±0.2°; and
12.5°±0.2°.

More specifically, the crystal A is a crystal form having characteristic peaks in the powder X-ray diffraction, at which the diffraction angle (2θ) is the following positions:
5.4°±0.2°;
7.7°±0.2°;
10.5°±0.2°;
12.5°±0.2°; and
14.5°±0.2°.

### (Crystal B)

The crystal B can be obtained, for example, by drying the crystal A.

The crystal B is a crystal form having characteristic peaks in the powder X-ray diffraction, at which the diffraction angle (2θ) is the following positions:
6.3°±0.2°;
10.1°±0.2°;
16.3°±0.2°; and
20.2°±0.2°.

More specifically, the crystal B is a crystal form having characteristic peaks in the powder X-ray diffraction, at which the diffraction angle (2θ) is the following positions:
6.3°±0.2°;
10.1°±0.2°;
13.7°±0.2°;
16.3°±0.2°; and
20.2°±0.2°.

The crystal B has a high solubility. For this reason, the crystal B can be said to have properties desired in the case where Compound (I) is used in tablets or the like.

Specifically, the crystal B can achieve a solubility at pH 6.9 of 50 µg/ml or more. Here, the solubility at pH 6.9 is measured by HPLC analysis. Specifically, the solubility at pH 6.9 can be measured in the method described below in Examples.

### (Crystal C)

The crystal C can be obtained, for example, through crystallization of Compound (I) by using acetonitrile, isopropyl acetate, and heptane as a crystallization solvent.

The crystal C is a crystal form having characteristic peaks in the powder X-ray diffraction, at which the diffraction angle (2θ) is the following positions:
6.8°±0.2°;
17.4°±0.2°; and
19.8°±0.2°.

More specifically, the crystal B is a crystal form having characteristic peaks in the powder X-ray diffraction, at which the diffraction angle (2θ) is the following positions:
6.8°±0.2°;
10.1°±0.2°;
12.7°±0.2°;
17.4°±0.2°; and
19.8°±0.2°.

The crystal C has physical properties favorable in terms of stability due to its high melting point.

### Examples

Hereinafter, Examples conducted by the present inventors will be described in order to describe the present invention in more details. However, the present invention should not be interpreted as being limited to Examples described below. Unless otherwise specified, as reagents used in Examples, either commercially available reagents or reagents prepared using methods known to those skilled in the art were used.

### Example 1: Step (a-1-1)

### Preparation of Compound 3-Br (N-(5-bromopyridin-2-yl)-3-oxobutanamide)

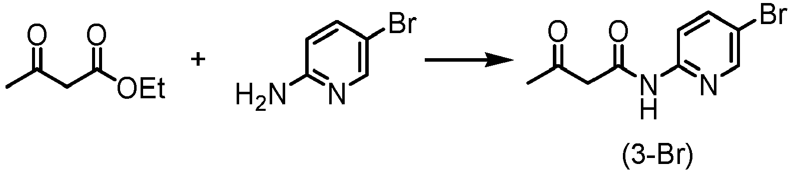

To a reaction vessel, 2-amino-5-bromopyridine (0.10 kg), toluene (0.90 L), ethyl acetoacetate (0.15 kg), and N,N-diisopropylethylamine (0.0037 kg) were added, and the mixture was stirred at 117°C for 14 hours. Then, the reaction liquid was cooled and crystals were precipitated. The precipitated crystals were washed with cooled toluene. After the washing, the crystals were dried by drying under reduced pressure. As a result, the dry crystals of the compound 3-Br (0.13 kg) were obtained.

The identification results of the dried crystals obtained were as follows.
¹H NMR (400MHz, DMSO-d₆) δ 10.75 (s, 1H), 8.42 (dd, J=2.4, 0.9Hz, 1H), 8.12-8.02 (m, 1H), 7.99 (dd, J=8.9, 2.5 Hz, 1H), 3.66 (s, 2H), 2.19(s, 3H)
¹³C NMR (101MHz, DMSO-d₆) δ 202.61, 166.16, 150.72, 148.51, 140.64, 114.98, 113.52, 52.06, 30.21
LC-MS(ESI, m/z): 257[M+H]⁺

### Example 2: Step (a-1-2)

### Preparation of Compound 4-Br ((Z)-N-(5-bromopyridin-2-yl)-3-(methylamino)but-2-enamide

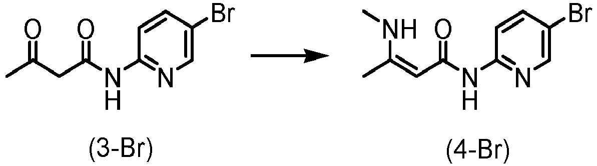

To a reaction vessel, the compound 3-Br (0.10 kg) prepared in Example 1 and methanol (0.5 L) were added. After that, at room temperature, a 9.8 Mol/L methylamine methanol solution (0.091 L) was added to the reaction solution, followed by stirring for 2 hours. After the reaction, the precipitated crystals were washed with methanol. After the washing, the crystals were dried by drying under reduced pressure. As a result, the dry crystals of the compound 4-Br (0.10 kg) were obtained.

The identification result of the obtained dry crystals was as follows.
¹H NMR (400MHz, DMSO-d₆) δ 9.74 (s, 1H), 9.17-8.96 (m, 1H), 8.28 (dd, J=2.6, 0.7Hz, 1H), 8.10 (dd, J=9.0, 0.7 Hz, 1H), 7.83 (dd, J=9.0, 2.6Hz, 1H), 4.75 (s, 1H), 2.86 (d, J=5.2Hz, 3H), 1.87 (s, 3H)
¹³C NMR (101MHz, DMSO-d₆) δ 168.52, 161.74, 152.39, 148.07, 139.80, 114.19, 111.01, 84.74, 29.14, 19.08
LC-MS (ESI, m/z): 270 [M+H]⁺

The crystals were determined to be in a Z form from a NOESY measurement result.

### Example 3: Step (a-1-3)

### Preparation of Compound 5-Br (3-(5-bromopyridin-2-yl)-1,6-dimethylpyrimidine-2,4(1H,3H)-dione)

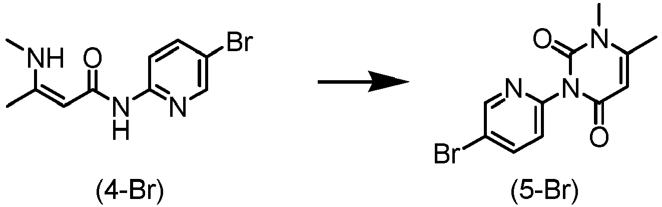

To a reaction vessel, the compound 4-Br (10.0 g) prepared in Example 2, tetrahydrofuran (200 mL), and N,N-diisopropylethylamine (13.6 mL) were added, followed by stirring at room temperature. During the stirring, phenyl chloroformate (8.41 mL) was added slowly dropwise. The reaction liquid was stirred for 2 hours. Then, diazabicycloundecene (12.7 mL) was added, followed by stirring at 70° for 18 hours. After that, the reaction liquid was cooled to 50°C. After the cooling, isopropanol was added. Then, ethyl acetate (200 mL) and activated carbon (5.01 g) were added, followed by stirring at 50°C for 2 hours. Then, the reaction liquid was cooled to room temperature and then filtered through Celite. The residue was washed with ethyl acetate (50 mL) three times, and the filtrate was concentrated under reduced pressure. Next, a mixture solvent of methyl tert-butyl ether and isopropanol was added to precipitate crystals. The precipitated crystals were washed with methyl tert-butyl ether. Further, the resultant crystals were dried under reduced pressure. As a result, the dry crystals of the compound 5-Br (6.10 g) were obtained.

The identification result of the obtained dry crystals was as follows.
¹H NMR (400MHz, DMSO-d₆) δ 8.72 (dd, J=2.5, 0.7Hz, 1H), 8.23 (dd, J=8.4, 2.5Hz, 1H), 7.40 (dd, J=8.4, 0.7Hz, 1H), 5.76 (d, J=0.9Hz, 1H), 3.33 (s, 3H), 2.32 (d, J=0.9Hz, 3H)
LC-MS (ESI, m/z): 296 [M+H]⁺

### Example 4: Step (a-2-1)

### Preparation of Compound 7 (1,6-dimethylpyrimidine-2,4(1H,3H)-dione)

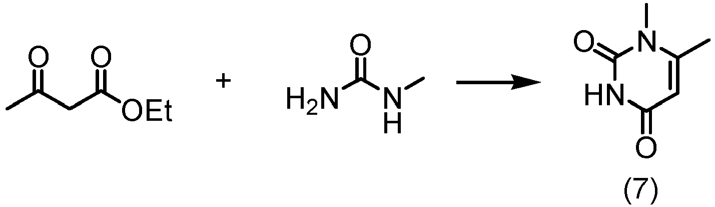

To a reaction vessel, 1-methylurea (1.0 kg), toluene (15 L), ethyl acetoacetate (3.51 kg), and p-toluenesulfonic acid monohydrate (0.26 kg) were added, and the mixture was stirred at 120°C for 40 hours. Then, the reaction liquid was cooled to precipitate crystals. Further, the precipitated crystals were washed with toluene. A mixture solvent of ethanol and water was added to the obtained crystals, followed by stirring. After the stirring, the precipitated crystals were washed with a mixture solvent of ethanol and water. Further, the resultant crystals were dried under reduced pressure to obtain dry crystals of the compound 7 (0.85 kg).

The identification result of the obtained dry crystals was as follows.
¹H NMR (400MHz, DMSO-d₆): δ 11.11 (s, 1H), 5.47 (s, 1H), 3.23 (s, 3H), 2.20 (s, 3H)
¹³C NMR (101MHz, DMSO-d₆) δ 162.54, 154.94, 151.79, 100.45, 30.16, 19.60
LC-MS (ESI, m/z): 141 [M+H]⁺

### Example 5: Step (a-2-2)

### Preparation of Compound 5-Br (3-(5-bromopyridine-2-yl)-E6-dimethylpyrimidine-2,4(1H, 3H)-dione)

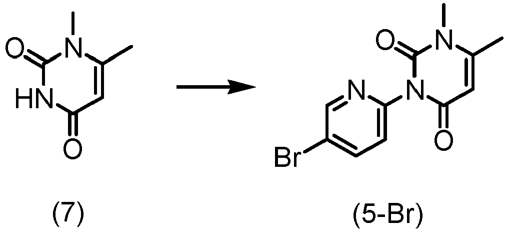

To a reaction vessel, the compound 7 (10.0 kg) synthesized in Example 4, N,N-dimethylformamide (50 L), 2,5-dibromopyridine (18.6 kg), copper(I) oxide (5.13 kg), and 2,4,6-collidine (20 L) were added, and the reaction solution was stirred at 110 °C for 16 hours. After that, the reaction liquid was cooled and dichloromethane (200 L) was added dropwise. Next, ammonia water (100 L) was added. Thereafter, the reaction solution was filtered through Celite. Further, the organic layer was sequentially washed once with water, twice with aqueous ammonia, twice with an aqueous citric acid solution, and twice with 10% brine. After the filtrate was concentrated, methyl tert-butyl ether (100 L) was added thereto, followed by stirring. Next, the precipitated crystals were washed with methyl tert-butyl ether. Further, the resultant crystals were dried under reduced pressure. As a result, the dry crystals of the compound 5-Br (12.06 kg) were obtained.

The identification result of the obtained dry crystals was as follows.
¹H NMR (400MHz, DMSO-d₆): δ 8.72 (d, J=2.0Hz, 1H), 8.23 (dd, J=8.4, 2.4Hz, 1H), 7.40 (d, J=8.4Hz, 1H), 5.76 (s, 1H), 3.32 (s, 3H), 2.31 (s, 3H)
LC-MS (ESI, m/z): 296 [M+H]⁺

### Example 6: Step (b)

### Preparation of Compound 10-Boc (isobutyl(S)-2-[(tert-butoxycarbonyl)amino]-3-[6-(3,4-dimethyl-2,6-dioxo-3,6-dihydropyrimidin-1(2H)-yl)pyridin-3-yl]propanoate)

### Step (b-1)

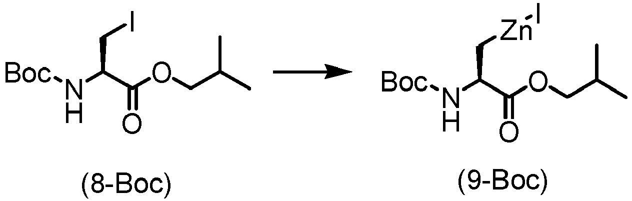

To a reaction vessel, N,N-dimethylformamide (240.0 L) and zinc (38.68 kg) were added, followed by stirring at 25°C for 15 minutes. Further, iodine (6.84 kg) was added, followed by stirring for 10 minutes. After that, a solution of a compound 8-Boc (isobutyl(R)-2-[(tert-butoxycarbonyl)amino]-3-iodopropanoate) (85.24 kg) dissolved in N,N-dimethylformamide (157.5 L) was added dropwise to the reaction vessel. The reaction liquid was stirred at 30°C for 10 minutes. As a result, a compound 9-Boc ((R)-{2-[(tert-butoxycarbonyl)amino]-3-isobutoxy-3-oxopropyl}zinc(II) iodide) was prepared.

### Step (b-2)

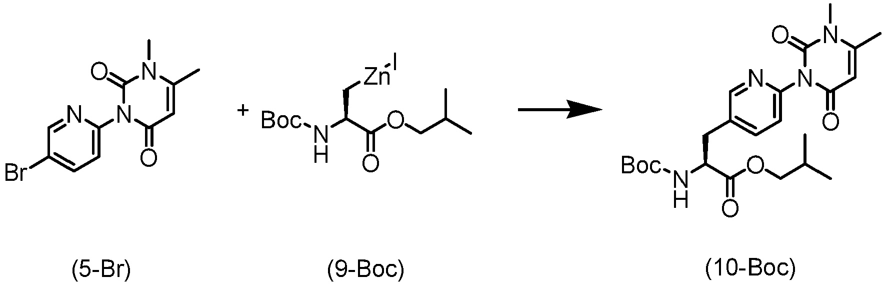

To a solution of the compound 9-Boc obtained in the step b-1, the compound 5-Br (40.0 kg) obtained in Example 5, palladium(II) acetate (1.06 kg), and S-Phos (5.8 kg) were added sequentially. Then, the reaction liquid was stirred at 55°C for 3 hours. Next, the reaction liquid was cooled and ethyl acetate and water were added thereto. Subsequently, the resultant liquid was filtered through Celite. Water was further added thereto, followed by stirring. Furthermore, after Celite filtration, the aqueous layer was removed by a liquid separation operation. Extraction from the removed aqueous layer was performed twice with ethyl acetate and the extract was mixed with the previously-obtained organic layer. After the mixing, the obtained liquid was washed four times with 10% brine. The organic layer was concentrated. After that, methyl tert-butyl ether (601.4 L) was added, followed by stirring to precipitate crystals. The precipitated crystals were washed with methyl tert-butyl ether. Further, the resultant crystals were dried under reduced pressure. As a result, the dry crystals of the compound 10-Boc (45.8 kg) were obtained.

The identification result of the obtained dry crystals was as follows.
¹H NMR (400MHz, DMSO-d₆): δ 8.42 (d, J=2.3Hz, 1H), 7.84 (dd, J=8.1, 2.4Hz, 1H), 7.44 (d, J=8.1Hz, 1H), 7.27 (d, J=8.1Hz, 1H), 5.73 (d, J=1.0Hz, 1H), 4.32-4.22 (m, 1H), 3.86 (dd, J=6.6, 1.1Hz, 2H), 3.32 (s, 3H), 3.12(dd, J=14.0, 4.7Hz, 1H), 2.96 (dd, J=14.1, 10.6Hz, 1H), 2.31 (d, J=0.9Hz, 3H), 1.87 (dt, J=13.3, 6.6Hz, 1H), 1.35 (s, 9H), 0.89 (d, J=6.7Hz, 6H).
LC-MS (ESI, m/z): 461 [M+H]⁺

### Example 7: Step (c-1)

### Preparation of Compound 12 (N,N'-[disulfandylbis(4,1-phenylene)]bis(2,2-dimethylpropanamide))

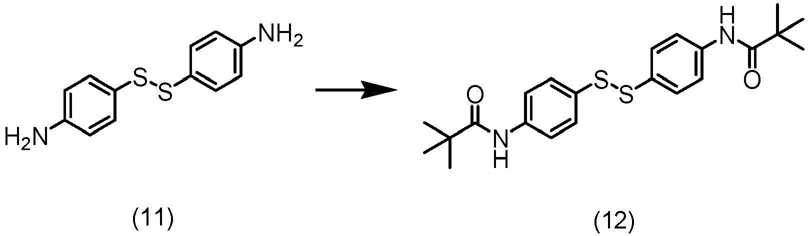

To a reaction vessel, a compound 11 (4,4'-dithiodianiline) (50.0 g), dichloromethane (750 mL), and triethylamine (61.1 g) were added. Pivaloyl chloride (60.7 g) was added while the temperature was kept at 15°C or lower, followed by stirring at room temperature for 1 hour. To the reaction solution, water (750 mL) was added and the mixture was stirred at room temperature for 1 hour. After that, precipitated crystals were filtered out. The obtained crystals were washed with water (100 mL). Further, the resultant crystals were dried at 50°C. As a result, the dry crystals of the compound 12 (78.0 g) were obtained.

The identification result of the obtained dry crystals was as follows.
¹H NMR (400MHz, DMSO-d₆): δ 9.31 (s, 2H), 7.71-7.63 (m, 4H), 7.46-7.37 (m, 4H), 1.21 (s, 18H)
¹³C NMR (101MHz, DMSO-d₆): δ 176.60, 139.62, 129.86, 129.49, 120.84, 39.21, 27.10
LC-MS (ESI,m/z): 417 [M+H]⁺

### Example 8: Step (c-2)

### Preparation of Compound 13 (4-pivalamidobenzenesulfonyl chloride)

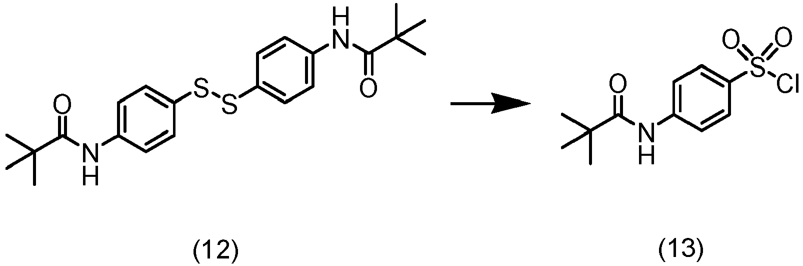

To a reaction vessel, the compound 12 (30.0 g) and acetic acid (240 mL) were added. With stirring at 20°C, a 12% NaClO aqueous solution prepared from NaClO·5H₂O (71.1 g, 432 mmol) was added slowly dropwise to the reaction vessel. After stirring at room temperature for 5 hours, 240 ml of water was added to the reaction liquid. Then, the reaction liquid was stirred at room temperature for 30 minutes, and precipitated crystals were filtered out. The obtained crystals were suspended in water (240 mL), followed by stirring at room temperature for 1 hour. The crystals were filtered out. Then, the crystals were washer with water (120 mL). Subsequently, the crystals were dried at 50°C. As a result, the dry crystals of the compound 13 (30.5 g) were obtained.

The identification result of the obtained dry crystals was as follows.
¹H NMR (400MHz, CDCl₃): δ 8.02-7.96 (m, 2H), 7.84-7.78 (m, 2H), 7.59 (s, 1H), 1.34 (s, 9H)
¹³C NMR (101MHz, CDCl₃): δ 177.05, 144.32, 138.75, 128.64, 119.54, 40.07, 27.48
LC-MS (ESI,m/z): 276 [M+H]⁺

### Example 9: Step (c-3)

### Preparation of Compound 15 (2,5-difluoro-4-((4-pivalamidophenyl)sulfonamido)benzoic acid)

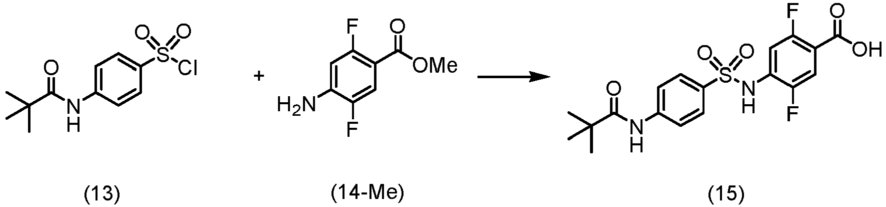

### Step (c-3-1)

To a reaction vessel, a compound 14-Me (methyl 4-amino-2,5-difluorobenzoate) (18 g), dichloromethane (180 mL), the compound 13 (28.9 g), and pyridine (38.7 mL) were added at 35°C or lower. After that, the reaction liquid was stirred at 35°C for 8 hours.

### Step (c-3-2)

After the reaction, an operation of adding tetrahydrofuran (90.0 mL) to the reaction liquid and concentrating the resultant liquid under reduced pressure until the solution volume became 72.0 mL was repeated three times. Tetrahydrofuran (90.0 mL) and methanol (90.0 mL) were added to the concentrated residue and the product was dissolved. Then, a 2M sodium hydroxide aqueous solution (221 mL) was added at 30°C or lower, followed by stirring at room temperature for 4 hours. After the reaction, the reaction liquid was washed twice with methyl tert-butyl ether (270 mL). To the obtained aqueous layer, 6M hydrochloric acid (63.0 mL) was added at 30°C or lower, followed by stirring at room temperature for two hours. The precipitated crystals were filtered out. Next, the crystals were washed with water (36.0 mL). Tetrahydrofuran (54.0 mL) and acetone (90.0 mL) were added to the obtained crystals. Further, water (216 mL) was added dropwise at 45°C with stirring. The stirring was performed at 45°C for 1 hour. After that, the resultant was cooled to 25°C and further stirred for 1 hour. The precipitated crystals were filtered out, and the crystals after the filtration were washed with water (72.0 mL). The obtained crystals were dried at 25°C for 1 hour. Subsequently, the crystals were further dried at 60°C for 8 hours. As a result, the dry crystals of the compound 15 (28.2 g) were obtained.

The identification result of the obtained dry crystals was as follows.
¹H NMR (400MHz, DMSO-d₆): δ 13.38 (s, 1H), 10.87 (s, 1H), 9.60 (s, 1H), 7.93-7.85 (m, 2H), 7.85-7.76 (m, 2H), 7.61 (dd, J=10.6, 6.6Hz, 1H), 7.24 (dd, J=11.8, 6.4Hz, 1H), 1.23 (s, 9H)
¹³C NMR (101MHz, DMSO-d₆) δ 177.59, 163.98, 163.94, 158.88, 156.35, 150.46, 148.04, 144.38, 133.18, 128.16, 120.28, 118.79, 118.77, 118.56, 110.62, 110.33, 39.90, 27.41
LC-MS (ESI, m/z): 413 [M+H]⁺

### Example 10: Preparation of Compound I in One-Pot Method (Steps (d) to (e))

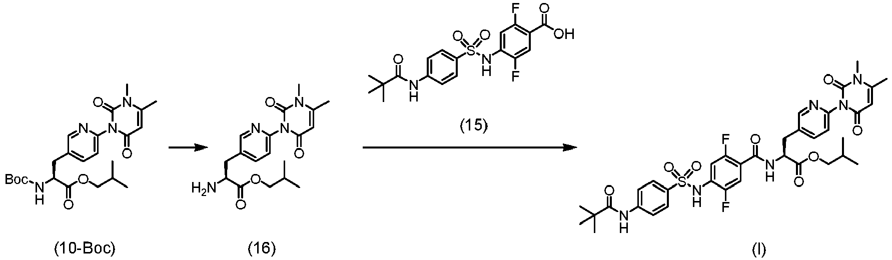

### Step (d-1)

To a reaction vessel, the compound 10-Boc (73.79 kg) prepared in Example 6, acetonitrile (900 L), sodium iodide (26.15 kg), and chlorotrimethylsilane (31.61 kg) were added, and the mixture was stirred at 40°C for 4 hours. Then, the reaction solution was cooled.

### Step (d-2)

Next, to the reaction solution obtained in the step (d-1), the compound 15 (59.94 kg) obtained in Example 9, 1-hydroxybenzotriazole monohydrate (24.47 kg), N-methylmorpholine (44.15 kg), and 1-ethyl-3-(3-dimethylaminopropyl) carbodiimide hydrochloride (30.53 kg) were added, and the mixture was stirred at 30°C for 1 hour. After the reaction, water (180 L) was added. Further, the reaction liquid was concentrated under reduced pressure. After that, ethyl acetate (839 L) and hydrochloric acid prepared with concentrated hydrochloric acid (63.0 L) and water (180 L) were added and the aqueous layer was re-extracted with the ethyl acetate. The organic layers were combined and were washed sequentially with water, twice with 8% aqueous sodium hydrogen carbonate solution, and twice with 10% brine. The organic layer was filtered and the filtrate was concentrated. Isopropanol (180 L) was added to the concentrated liquid, and the resultant liquid was re-concentrated.

### Step (e)

Further, isopropanol (300 L) was added, followed by stirring at 35°C. After 0.600 kg of seed crystals were inoculated into the reaction liquid, the resultant liquid was stirred at the same temperature for 8 hours. Subsequently, the reaction liquid was cooled to 25°C. After the cooling, isopropanol (420 L) was added, and the resultant liquid was stirred at the same temperature for 5 hours. The precipitated crystals were washed with isopropanol. As a result, the crystal A of Compound I (isobutyl(S)-2-(2,5-difluoro-4-((4-pivalamidophenyl)sulfonamido)benzamide)-3-(6-(3,4-dimethyl-2,6-dioxo-3,6-dihydropyrimidin-1(2H)-yl)pyridin-3-yl)propanoate) was obtained.

Note that the seed crystals were obtained by stirring Compound I (100 mg) obtained by the method described in Patent Literature 1 (which is an amorphous substance obtained by purification with high-performance liquid chromatography after synthesis, and then freeze-drying) in isopropanol (1.5 mL) for one week.

Then, the crystal A was dried under reduced pressure. Thus, the dry crystal B of Compound I (88.22 kg) was obtained.

### Example 11: Preparation of Crystal C

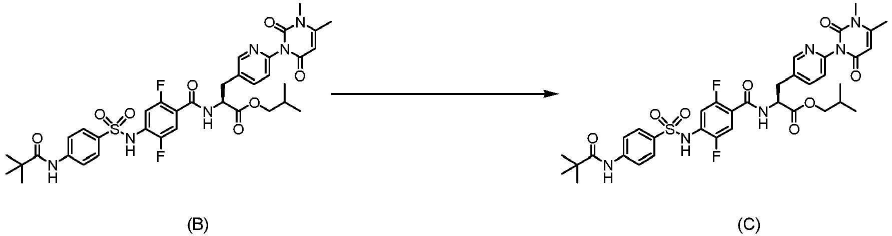

To a reaction vessel, the crystal B of Compound I (50.0 g) and acetonitrile (75 mL) were added and the mixture was stirred at 40°C. Then, after the mixture was filtered for clarifying, the resultant was heated to 75°C. Next, isopropyl acetate (80 mL) was added to the reaction vessel. After that, while the acetonitrile was removed by distillation under reduced pressure, isopropyl acetate (125 mL) was added. Thereafter, 0.10 g of seed crystals were inoculated. Note that the seed crystals were obtained by stirring the crystal B of Compound I (20 g) in isopropyl acetate (100 mL) at 90°C for two hours, and adding heptane (100 mL) thereto, followed by cooling to 25°C and filtration.

Next, while the acetonitrile was removed by distillation under reduced pressure, isopropyl acetate (375 mL) was gradually added. The resultant was stirred at 75°C for 15 hours. Then, heptane (100 mL) was added and the mixture was cooled to 10°C and stirred for 5 hours. The precipitated crystals were washed with heptane. After the washing, the crystals were dried under reduced pressure. As a result, the dry crystal C of Compound I (48.2 g) was obtained.

### Example 12-1: Measurement of Powder X-ray Diffraction Patterns

Regarding the crystals A and B obtained in Example 10 and the crystal C obtained in Example 11, powder X-ray diffraction patterns were measured. The conditions for measuring the powder X-ray diffraction patterns were set as follows.

### (Measurement of Powder X-ray Diffraction Pattern)

A powder X-ray diffraction measurement was performed on the crystals obtained in each Example according to the powder X-ray diffraction measurement method described in General Tests, Processes and Apparatus of the Japanese Pharmacopoeia. The measurement conditions were set as follows.

### (Apparatus)

MiniFlex 600-C manufactured by Rigaku Corporation

### (Operation Method)

Measurement method: Reflection method
Wavelength used: CuKα ray
Tube current: 15 mA
Tube voltage: 40 kV
X-ray incident angle: 1.25°
Step width: 0.01°
Scan range: 2 to 60°
Detector: D/teX Ultra2

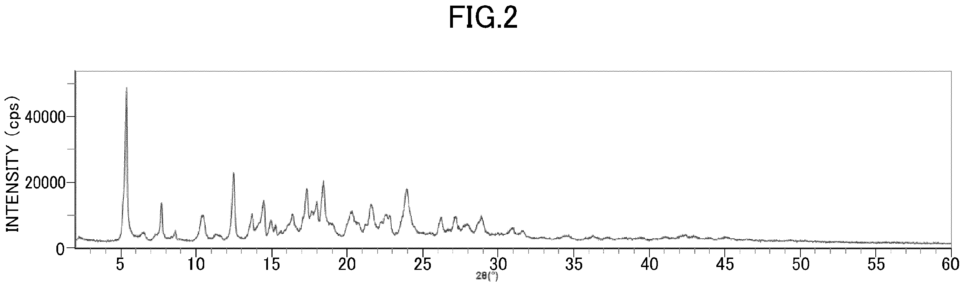

Fig. 2 shows a powder X-ray diffraction pattern of the crystal A. The characteristic peaks were observed at 5.4°, 10.5°, and 12.5°, and more specifically, at 5.4°, 7.7°, 10.5°, 12.5°, and 14.5°.

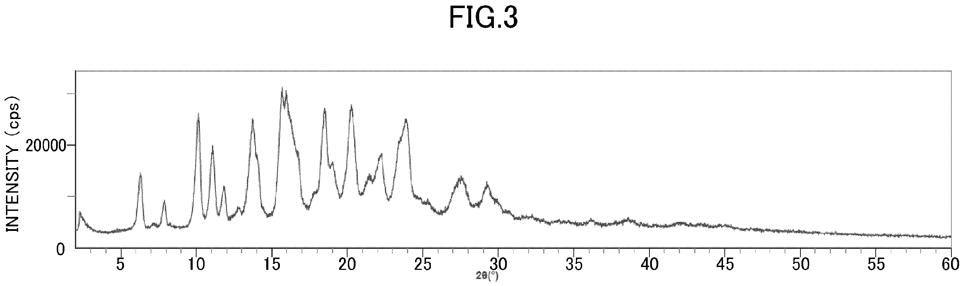

Fig. 3 shows a powder X-ray diffraction pattern of the crystal B. The characteristic peaks were observed at 6.3°, 10.1°, 16.3°, and 20.2°, and more specifically, at 6.3°, 10.1°, 13.7°, 16.3°, and 20.2°.

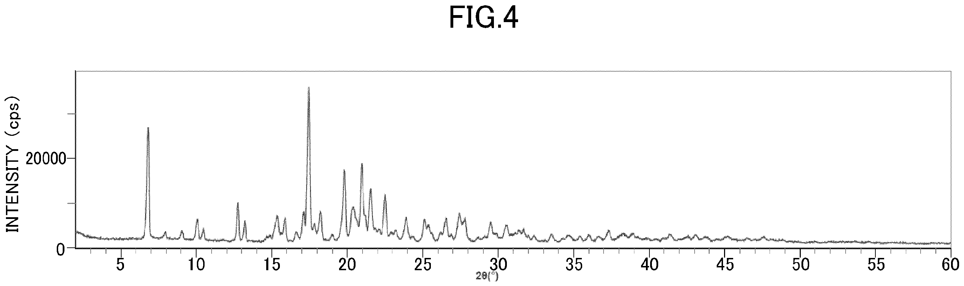

Fig. 4 shows a powder X-ray diffraction pattern of the crystal C. The characteristic peaks were observed at 6.8°, 17.4°, and 19.8°, and more specifically, at 6.8°, 10.1°, 12.7°, 17.4°, and 19.8°.

### Example 12-2: Measurement of Solubility at pH 6.9

Regarding the crystals B and C, the solubility at pH 6.9 was measured. Specifically, a sample was excessively added to a second dissolution test solution (pH 6.9) (manufactured by Kanto Chemical Co., Inc.), and the mixture was shaken at 20°C for 24 hours to produce a saturated solution. After the undissolved sample was removed with a filter, the amount of the compound in the filtrate was determined by HPLC, and the solubility (µg/ml) was calculated.

As a result, the solubility at pH 6.9 of the crystal B was 150 µg/ml. The solubility at pH 6.9 of the crystal C was 25.0 µg/ml. This indicates that the crystal B is more suitable for application requiring high solubility.

### Example 12-3: Measurement of Moisture Absorption

Regarding the crystals B and C, the moisture absorption was measured by using an automatic gas and vapor adsorption analyzer (BELSORP Aqua3 manufactured by MicrotracBEL Corp.)

As a result, the moisture absorption of the crystal B was at maximum 1.35%. On the other hand, the moisture absorption of the crystal C was at maximum 0.24%. This result confirmed that both crystal forms pose no particular problem in terms of moisture absorption when used as pharmaceutical compositions. However, the crystal C has lower moisture absorption and therefore is more preferable in this respect.

### Example 12-4: Measurement of Melting Point

Regarding the crystals B and C, the melting point was measured by using a thermal analyzer (8225A600 manufactured by Rigaku Corporation). As a result, the melting point of the crystal B was 151.9°C. The melting point of the crystal C was 182.5°C. This indicates that the crystal C has a higher melting point and therefore is more preferable in terms of stability.

### Example 13: Preparation of Crystals B by Using Ethanol Solvent

To a reaction vessel, the crystal B of Compound I (5.93 g) and ethanol (24 mL) were added and the mixture was stirred at 70°C. After that, the mixture was cooled to 45°C and 0.040 g of seed crystals were inoculated therein. Note that the seed crystals were obtained according to the same procedure as in the seed crystals used in Example 10.

Next, the resultant mixture was cooled to 30°C over 3 hours, and was cooled to 0°C over 3 hours and then stirred at the same temperature for 5 hours. The precipitated crystals were washed with ethanol. As a result, the crystals of Compound I were obtained. The crystal form of the obtained crystals was checked with a powder X-ray diffraction pattern, and found to be the crystal A.

Then, the crystal A was dried under reduced pressure. As a result, the dry crystals of Compound I (5.25 g) were obtained. The crystal form of the obtained crystals was checked with a powder X-ray diffraction pattern, and found to be the crystal B.

### Example 14: Preparation of Compound I in Catalytic Reduction Method (Steps (d) to (e))

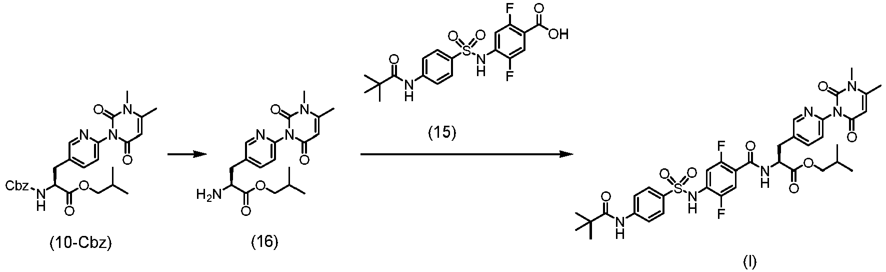

### Step (d-1)

To a reaction vessel, a compound 10-Cbz (isobutyl(S)-2-(((benzyloxy)carbonyl)amino)-3-(6-(3,4-dimethyl-2,6-dioxo-3,6-dihydropyrimidin-1 (2H)-yl)pyridin-3-yl)propanoate) (1.00 g), tetrahydrofuran (7.6 mL), and 10% palladium on carbon (0.10 g) were added, and the mixture was stirred in a hydrogen atmosphere at 60°C for 22 hours. The reaction liquid was cooled. After the cooling, the reaction liquid was filtered through Celite to remove the palladium on carbon. Further, the Celite was washed with tetrahydrofuran. As a result, the compound 16 solution was obtained.

### Step (d-2)

To the compound 16 solution obtained in the step (d-1) without the product isolated, the compound 15 (0.76 g), 1-hydroxybenzotriazole monohydrate (0.31 g), N-methylmorpholine (1.04 g), and 1-ethyl-3-(3-dimethylaminopropyl)carbodiimide hydrochloride (0.39 g) were added, and the mixture was stirred at 30°C for 15 hours. After the reaction, water (2.3 mL) was added. Next, the reaction liquid was concentrated under reduced pressure. Then, ethyl acetate (11.4 mL) and water (7.6 mL) were added thereto, and the aqueous layer was extracted with ethyl acetate. Ethyl acetate (11.4 mL) was added to the aqueous layer and the aqueous layer was extracted again with ethyl acetate. The organic layers were combined and washed sequentially with 1M hydrochloric acid, an 8% aqueous sodium hydrogen carbonate solution, and 10% brine. The organic layer was concentrated. Isopropanol (2.3 mL) was added to the concentrated liquid, and the resultant liquid was concentrated again.

### Step (e)

Further, isopropanol (3.0 mL) was added thereto, followed by stirring at 35°C. After 7.6 mg of seed crystals were inoculated into the reaction liquid, the resultant liquid was stirred at the same temperature. As the seed crystals, the same seed crystals as those used in Example 10 were used. After the reaction liquid was cooled to 20°C, isopropanol (6.1 mL) was added and the mixture was stirred at the same temperature. The precipitated crystals were washed with isopropanol. Further, the crystals were dried under reduced pressure. As a result, the dry crystals of Compound I (1.10 g) were obtained.

### Example 15: Preparation of Compound I in One-Pot Method (Steps (d) to (e))

### Step (d-1)

To a reaction vessel, the compound 10-Boc (4.91 g) prepared in Example 6, acetonitrile (60 mL), sodium iodide (1.75 g), and chlorotrimethylsilane (2.11 g) were added, and the mixture was stirred at 40°C for 4 hours. Then, the reaction solution was cooled.

### Step (d-2)

Subsequently, to the reaction solution obtained in the step (d-1), the compound 15 (4.00 g) obtained in Example 9, 1-hydroxybenzotriazole monohydrate (1.63 g), N-methylmorpholine (2.94 g), and 1-ethyl-3-(3-dimethylaminopropyl)carbodiimide hydrochloride (2.05 g) were added, and the mixture was stirred at 30°C for 1 hour. After the reaction, water (12 mL) was added. Further, the reaction liquid was concentrated under reduced pressure. Then, ethyl acetate (56 mL) and 1N hydrochloric acid (40 mL) were added and the aqueous layer was re-extracted with ethyl acetate. The organic layers were combined and were washed sequentially with water, twice with an 8% aqueous sodium hydrogen carbonate solution, and with 10% brine. The organic layer was filtered and the filtrate was concentrated. Ethanol (12 mL) was added to the concentrated liquid and the resultant liquid was concentrated again.

### Step (e)

Further, ethanol (24 mL) was added thereto, followed by stirring at 45°C. After the reaction liquid was cooled to 30°C, 0.040 g of seed crystals were inoculated into the reaction liquid, and thereafter the resultant liquid was stirred at the same temperature for 1.5 hours. Then, the reaction liquid was cooled to 0°C over 3 hours, and then was stirred at the same temperature for 13 hours. The precipitated crystals were washed with ethanol. As a result, the crystal A of Compound I (isobutyl(S)-2-(2,5-difluoro-4-((4-pivalamidophenyl)sulfonamido)benzamide)-3-(6-(3,4-dimethyl-2,6-dioxo-3,6-dihydropyrimidin-1(2H)-yl)pyridin-3-yl)propanoate) was obtained.

Note that the seed crystals were obtained by stirring Compound I (100 mg) obtained by the method described in Patent Literature 1 (which is an amorphous substance obtained by purification with high-performance liquid chromatography after synthesis, and then freeze-drying) in isopropanol (1.5 mL) for one week.

Then, the crystal A was dried under reduced pressure. As a result, the dry crystal B of Compound I (5.26 g) was obtained.

### Comparative Example 1: Compound 16-HCl

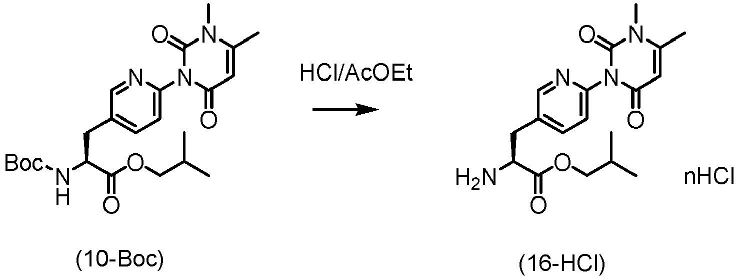

In reference to the description on page 56 in Patent Literature 1 (International Publication No. WO2017/135472), the compound 10-BoC was deprotected. Specifically, the compound 10-BoC was dissolved in ethyl acetate and the reaction liquid was cooled to 5°C. After that, a hydrochloric acid/ethyl acetate solution was added dropwise thereto, followed by stirring at 20°C for 16 hours. After that, the solid was filtered out and dried under reduced pressure to obtain a compound 16-HCl (a hydrochloric acid salt of the compound 16). The obtained compound was initially white powder, but when left in an environment of 60%RH and 25°C, was completely deliquesced within 24 hours.

From this result, it was found that the compound 16-HCl is difficult to be handled and is unsuitable for use in the next process during mass production. In contrast, it can be understood that the methods in Examples 10 and 14 are superior in productivity because there is no need to prepare a deliquescent compound such as the compound 16-HCl.

## Claims

1. A method for producing a compound of the following Formula (I) or a pharmaceutically acceptable salt thereof: 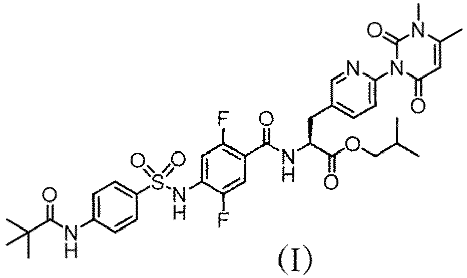 the method comprising:
a step (d-1) of preparing a compound 16 solution by deprotecting a compound 10-R₃ in a solvent according to the following scheme (d-1): 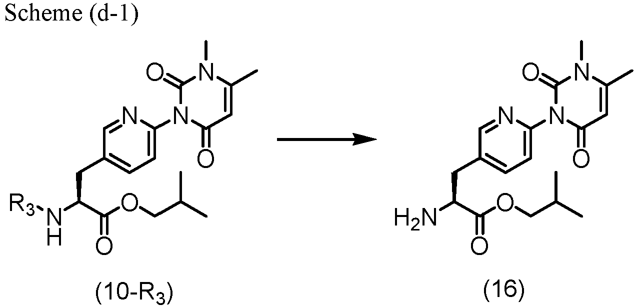 (wherein R₃ denotes a protecting group for amino group); and
a step (d-2) of adding a compound 15 to the compound 16 solution without the compound 16 isolated, and thereby synthesizing the compound of Formula (I) in the presence of a condensing agent according to the following scheme (d-2):

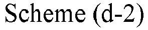

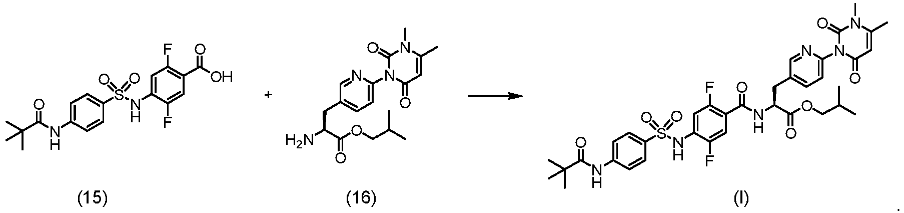

2. The method according to claim 1, wherein the step (d-1) and the step (d-2) are performed in a one-pot manner.

3. The method according to claim 1 or 2, wherein the step (d-1) includes a step of deprotecting the compound 10-R₃ in the presence of TMSX1 (wherein X1 denotes a halogen atom or triflate).

4. The method according to claim 2 or 3, wherein the solvent in the step (d-1) includes at least one selected from the group consisting of ketone solvents, nitrile solvents, and halogenated solvents.

5. The method according to claim 1, wherein the step (d-1) includes a step of deprotecting the compound 10-R₃ through catalytic reduction.

6. The method according to claim 5, wherein
the step of deprotecting through catalytic reduction includes a step of deprotecting the compound 10-R₃ in the presence of a catalyst,
the method for producing further comprises a step of removing the catalyst from the compound 16 solution, and
in the step (d-2), the compound 15 is added to the compound 16 solution from which the catalyst has been removed.

7. The method according to claim 5 or 6, wherein the solvent in the step (d-1) includes tetrahydrofuran.

8. The method according to any one of claims 1 to 7, further comprising a step (e) of crystallizing the compound of Formula (I).

9. The method according to claim 8, wherein the step (e) of crystallizing includes a step of crystallizing the compound of Formula (I) in a solvent including at least one selected from the group consisting of water, alcohols having 1 to 4 carbon atoms, acetic acid esters having 3 to 6 carbon atoms, ether solvents having 2 to 10 carbon atoms, and aromatic hydrocarbons.

10. The method according to claim 8 or 9, wherein the step (e) of crystallizing includes a step of crystallizing the compound of Formula (I) in a solvent including isopropanol.

11. The method according to any one of claims 8 to 10, further comprising a step of drying the crystallized compound of Formula (I).

12. The method according to any one of claims 1 to 11, further comprising a step (b-2) of synthesizing the compound 10-R₃ by a reaction of a compound 5-X with a compound 9-R₃ according to the following scheme (b-2): 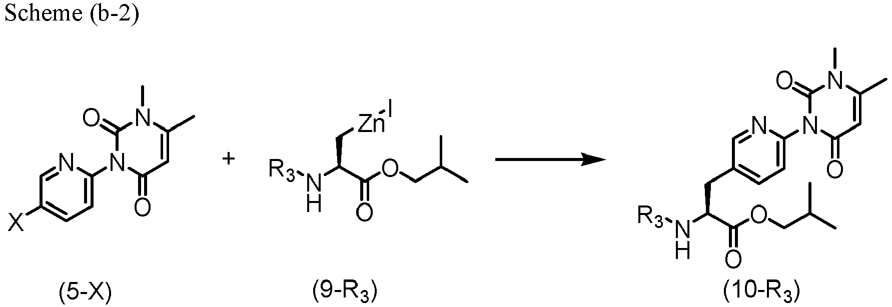 (wherein X denotes a halogen atom or triflate and R₃ denotes a protecting group for amino group).

13. The method according to claim 12, wherein X is a bromine atom.

14. The method according to claim 12 or 13, further comprising, before the step (b-2), a step (b-1) of synthesizing the compound 9-R₃ by a reaction of a compound 8-R₃ with zinc and iodine according to the following scheme (b-1):

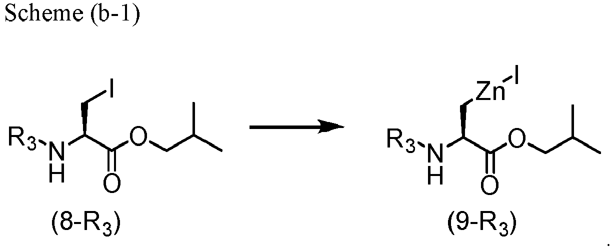

15. The method according to any one of claims 12 to 14, further comprising a step (a-1) of synthesizing the compound 5-X according to the following scheme (a-1): 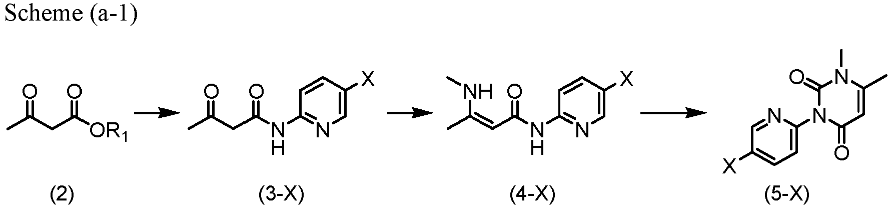 (wherein X denotes a halogen atom or triflate and R₁ denotes an alkyl group having 1 to 6 carbon atoms), wherein
the step (a-1) includes
a step of synthesizing a compound 3-X by a reaction of a compound 2 with a 2-aminopyridine derivative,
a step of synthesizing a compound 4-X by a reaction of the compound 3-X with methylamine, and
a step of synthesizing the compound 5-X by a reaction of the compound 4-X with a chloroformic acid ester.

16. The method according to any one of claims 12 to 14, further comprising a step (a-2) of synthesizing the compound 5-X according to the following scheme (a-2): 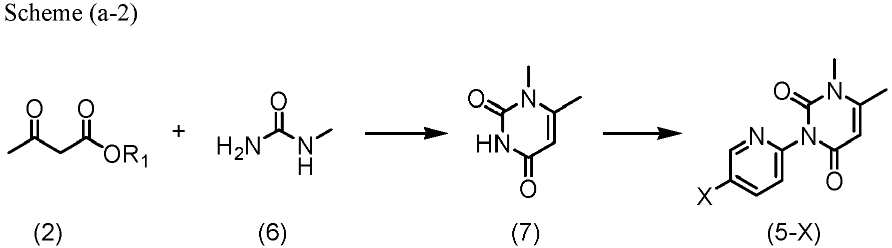 (wherein X denotes a halogen atom or triflate and R₁ denotes an alkyl group having 1 to 6 carbon atoms), wherein
the step (a-2) includes
a step of synthesizing a compound 7 by a reaction of a compound 2 with a compound 6, and
a step of synthesizing the compound 5-X by coupling the compound 7 with a pyridine derivative.

17. The method according to any one of claims 1 to 16, further comprising a step (c-3) of synthesizing the compound 15 by a reaction of a compound 13 with a compound 14-R₂ to form a sulfonamide, followed by hydrolysis with alkali according to the following scheme (c-3): 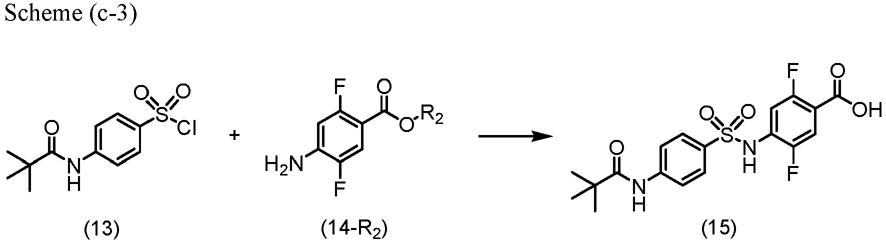 (wherein R₂ denotes an alkyl group having 1 to 6 carbon atoms).

18. The method according to claim 17, further comprising, after the step (c-3), a step of crystallizing the compound 15.

19. The method according to claim 17 or 18, further comprising a step of synthesizing the compound 13 by a reaction of a compound 12 with sodium hypochlorite under an acidic condition according to the following scheme (c-2):

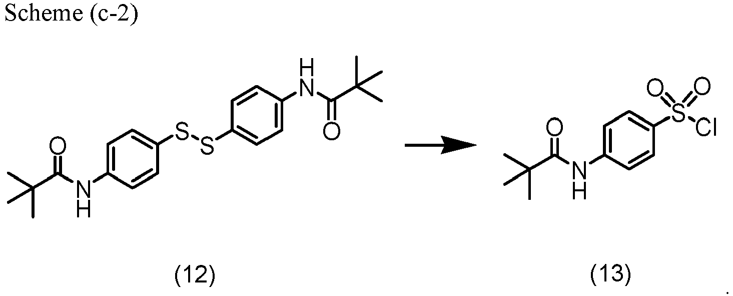

20. The method according to claim 19, further comprising a step (c-1) of synthesizing the compound 12 by a reaction of a compound 11 with a pivaloylation agent according to the following scheme (c-1):

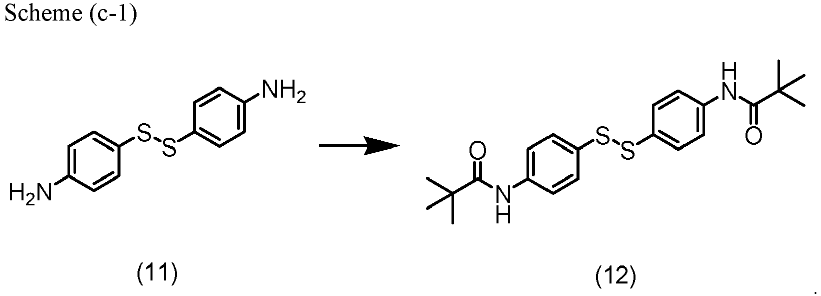

21. A compound represented by the following formula or a salt thereof: 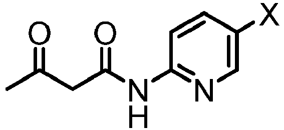 (wherein X denotes a halogen atom or triflate).

22. A compound represented by the following formula or a salt thereof: 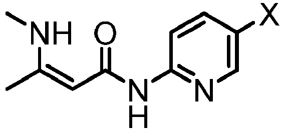 (wherein X denotes a halogen atom or triflate).

23. A crystal of a compound of the following Formula (I) or a pharmaceutically acceptable salt thereof:

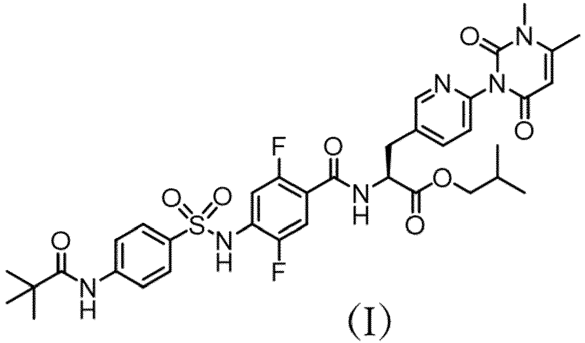

24. The crystal according to claim 23, wherein the crystal is obtained in a method including a step of crystallizing the compound of Formula (I) in a solvent including at least one selected from the group consisting of water, alcohols having 1 to 4 carbon atoms, acetic acid esters having 3 to 6 carbon atoms, ether solvents having 2 to 10 carbon atoms, and aromatic hydrocarbons.

25. The crystal according to claim 24, wherein the solvent includes isopropanol.

26. The crystal according to claim 24 or 25, which is obtained by drying a crystallized product after the step of crystallizing.

27. The crystal according to any one of claims 23 to 26, wherein a solubility at pH 6.9 is 50 µg/ml or more.

28. The crystal according to any one of claims 23 to 27, which is a solvate with ethanol.

29. The crystal according to any one of claims 23 to 27, which is a solvate with isopropanol.

30. The crystal according to any one of claims 23 to 29, which is a crystal B having peaks in powder X-ray diffraction, at which a diffraction angle (2θ) is
6.3°±0.2°,
10.1°±0.2°,
13.7°±0.2°,
16.3°±0.2°, and
20.2°±0.2°.

31. The crystal according to any one of claims 23 to 29, which is a crystal A having peaks in powder X-ray diffraction, at which a diffraction angle (2θ) is
5.4°±0.2°,
7.7°±0.2°,
10.5°±0.2°,
12.5°±0.2°, and
14.5°±0.2°.

32. The crystal according to any one of claims 23 to 29, which is a crystal C having peaks in powder X-ray diffraction, at which a diffraction angle (2θ) is
6.8°±0.2°,
10.1°±0.2°,
12.7°±0.2°,
17.4°±0.2°, and
19.8°±0.2°.
